# EUROPEAN PATENT APPLICATION

(11) **EP 4 253 423 A1**
(43) Date of publication of application: **04.10.2023**
(21) Application number: 21868731.7
(22) Date of filing: 17.09.2021
(51) Int. Cl.: C07K 16/46, A61P 35/00, A61K 39/395, C12N 1/21, C12N 15/13, C12N 5/10

(54) **BISPECIFIC RECOMBINANT PROTEIN AND USE THEREOF**

(30) Priority: 17.09.2020 CN 202010977716; 12.08.2021 CN 202110933105
(71) Applicant: Shanghai Lyn-Crest Enterprise Management Co., Ltd., Shanghai 201304 (CN)
(72) Inventor: SONG, Liping, Shanghai 201318 (CN); WANG, Suqin, Shanghai 201318 (CN)
(74) Representative: Dehns
(86) International application number: PCT/CN2021/119169
(87) International publication number: WO 2022/057909

(57) **Abstract**

A bispecific recombinant protein, comprising a first functional binding fragment, a second functional binding fragment, and an Fc region. The first functional binding fragment targeting an antigen of interest of the bispecific recombinant protein comprises an antigen-binding fragment, wherein the C-terminus of the CL domain or the C-terminus of the CH1 domain in the antigen-binding fragment is directly connected to or is connected via a linker to the second functional binding fragment having the functions of immunoregulation and/or metabolic regulation and/or endocrine regulation. Further disclosed is the use of the bispecific recombinant protein in the preparation of a drug for treating tumors, autoimmune diseases, infectious diseases, sepsis, graft-versus-host diseases, metabolic disorders and endocrine disorders. The recombinant protein can improve the targeting for the target of the second functional binding fragment of the bispecific recombinant protein and can also reduce toxic side effects caused by the non-target protein containing the second functional binding fragment generated during the preparation process targeting non-target organs, tissues and cells.

## Description

The present application claims the priorities of Chinese Patent Application No. 202010977716.2 filed on September 17, 2020, and Chinese Patent Application No. 202110933105.2 filed on August 12, 2021. The contents of the Chinese Patent Applications are incorporated herein by reference in their entireties.

### TECHNICAL FIELD

The present disclosure belongs to the field of biomedicine, in particular to a bispecific recombinant protein and use thereof.

### BACKGROUND

Immunity is a physiological function of the living organisms. Relying on this function to identify "sel" and "non-self" components, antigenic substances (such as bacteria, etc.) entering the human body or damaged cells and tumor cells generated by the living body itself are thereby destroyed and rejected, the occurrence and development of tumors are resisted, so as to maintain the health of the living organisms. However, in some cases, mutated cells can escape from the immune surveillance of the body through various mechanisms, rapidly proliferate in the body, and form tumors. The human body also needs immunosuppressive regulation to avoid abnormal immune function or continuous activation of immune, which results in organ damage such as autoimmune diseases, sepsis, and graft-versus-host disease (GVHD), etc., through immune inflammation, cytokine storm, and immune rejection of allografts.

Tumor immunotherapy is a treatment method to control and eliminate tumors by restarting and maintaining the tumor-immune cycle and restoring the body's normal anti-tumor immune response. Blocking these immune evasion mechanisms, or "promoting" immune activation by regulating and controlling the mechanisms of immunoregulation and immune activation is a common method of tumor immunotherapy at present, such as anti-PD-1 antibodies (e.g., nivolumab, pembrolizumab), anti-PD-L1 antibodies (e.g., durvalumab, atezolizumab), anti-CD47 antibodies/fusion proteins (e.g., magrolimab, TTI-621).

Autoimmune diseases refer to the diseases caused by the body's immune response to self-antigens, resulting in damage to its own tissues, comprising organ-specific autoimmune diseases and systemic autoimmune diseases. Wherein, organ-specific autoimmune diseases mainly comprise chronic lymphatic cellular thyroiditis, hyperthyroidism, insulin-dependent diabetes mellitus, myasthenia gravis, ulcerative colitis, pernicious anemia with chronic atrophic gastritis, Goodpasture's syndrome, pemphigus vulgaris, pemphigoid, primary biliary cirrhosis, multiple cerebrospinal sclerosis, acute idiopathic polyneuritis, etc. Systemic autoimmune diseases commonly comprise systemic lupus erythematosus, rheumatoid arthritis, systemic vasculitis, scleroderma, pemphigus, dermatomyositis, mixed connective tissue disease, autoimmune hemolytic anemia, autoimmune thyroid disease, ulcerative colitis, etc.

Sepsis is an acute systemic infection that occurs when various pathogenic bacteria invade the blood circulation, grow and multiply in the blood, and produce toxins. The clinical manifestations are generally acute onset, chills, high fever, shortness of breath, tachycardia, rash, swelling and pain in joint, hepatosplenomegaly, and change in mental status and consciousness, etc. Severe cases may develop acute organ dysfunction, and further aggravation may develop into septic shock, disseminated intravascular coagulation (DIC) and multiple organ failure. Sepsis is one of the most common causes of death in intensive care units. On the one hand, pathogenic bacteria or opportunistic pathogens multiply rapidly in the blood and cannot be eliminated by the immune system in time, resulting in acute systemic infection. On the other hand, toxins secreted by these bacteria, such as lipopolysaccharide (LPS), can activate macrophages and cause the release of inflammatory factors such as TNF, IL-1β, HMGB1, etc., resulting in a systemic inflammatory response, further aggravating the condition of sepsis, even endangering the life.

Graft-versus-host disease (GVHD) is due to T lymphocytes in donor allografts after transplantation, through a series of "cytokine storm" stimuli initiated by the recipient, greatly enhances its immune response to the recipient's antigens and initiate cytotoxic attacks to the recipient target cells. The incidence rate of acute graft-versus-host disease is 30% to 45%.

A large number of studies have shown that immune checkpoints or their ligands and cytokines play a very important role in disease prevention and treatment.

Immune checkpoints refer to a series of molecules that are expressed on immune cells and can regulate the degree of immune activation. Immune checkpoints and their ligands play an important role in regulating autoimmunity. The abnormal expression and function of immune checkpoint molecules is one of the important reasons for the occurrence of many diseases. For example, if the inhibitory immune checkpoint molecules are overexpressed or their function is too strong, the immune function is suppressed, the immunity of the body is low, and people are easily affected by infections, tumors and other diseases; on the contrary, if the immunosuppressive function of inhibitory immune checkpoint molecules is too weak, the immune function of the body will be abnormally active. Tumor cells can also express some substances to activate inhibitory immune checkpoints. Once activated, the latter is like stepping on the "brake", preventing antigens from being presented to T cells and blocking antigen presentation process in the tumor-immune cycle, and thereby inhibiting the immune function of T cells, allowing tumor cells to escape from surveillance and survive. Common immune checkpoints and their ligands such as PD-1/PD-L1, LAG-3/MHCII, CTLA-4/B7-1 or B7-2, TIM-3/Galectin-9, SIRPα/CD47, TIGIT/Nectin2 or Nectin3, BTLA/HVEM, etc.

PD-1 (programmed death receptor-1) is an important immunosuppressive molecule and belongs to the immunoglobulin superfamily. The binding of PD-1 and PD-L1 initiates the programmed death of T cells and enables tumor cells to obtain immune escape. Therefore, a large number of clinical research results have proved that blocking the immunoregulatory effect generated by the interaction between PD-1 and PD-L1 is of great significance in anti-tumor, anti-infection, anti-autoimmune diseases and organ transplantation survival.

LAG-3 (Lymphocyte-activation gene 3) belongs to the immunoglobulin superfamily and consists of three parts: extracellular region, transmembrane region and cytoplasmic region. Inhibition of LAG-3 can allow T cells to regain cytotoxicity and thus enhance their killing effect on tumors, and can also reduce regulatory T cells' function of suppressing immune responses.

CTLA-4 (cytotoxic T lymphocyte-associated antigen-4), also known as CD152, is a leukocyte differentiation antigen and a transmembrane receptor on T cells and shares the B7 molecular ligand with CD28. CTLA-4 binds to CD80 and CD86 (also known as B7-1 and B7-2) with greater affinity and avidity than CD28, transmits inhibitory signals to T cells, and participates in the negative regulation of immune responses.

TIM-3, also known as HAVCR2, belongs to the TIM gene family. As an immune checkpoint of negative regulation, TIM-3 exists in different types of immune cells, comprising T cells, regulatory T cells (Tregs), dendritic cells (DCs), B cells, macrophages, natural killer cells (NKs) and mast cells. TIM-3 is a type I membrane protein consisting of 281 amino acids. It consists of an extracellular domain, a single transmembrane domain and a C-terminal cytoplasmic tail. TIM-3 and its ligand Gal-9 suppress tumor immunity by negatively regulating the immunity of T cell. Linking of the TIM-3 IgV domain and Gal-9 may terminate the Th1 immune response. TIM-3 may induce immune tolerance and is associated with asthma, food allergies, and autoimmune diseases such as multiple sclerosis and rheumatoid arthritis. TIM-3 can also suppress the immune responses of T cells and relate to immune exhaustion leading to chronic viral infections.

CD47 is a transmembrane glycoprotein that belongs to the immunoglobulin superfamily and is expressed on the surface of almost all cells, including red blood cells. The ligands of CD47 comprise integrin, thrombospondin-1, and signal regulatory protein (SIRP). CD47 has a variety of biological functions, comprising migration of cells, activation of T cells and dendritic cells, development of axons, etc. In addition, CD47 can inhibit the phagocytosis of macrophages by interacting with SIRPα. In this way, CD47 transmits a so-called "Don't eat me" signal, which can protect normal cells such as red blood cells, B cells, and T cells from being engulfed by macrophages.

SIRP-alpha (SIRPα) is a typical inhibitory immune receptor in the SIRP family and can interact with the transmembrane glycoprotein CD47, which is widely present in the body, and transmit inhibitory signals. The SIRPα fusion protein can block the binding of CD47 and SIRP-alpha that is on the surface of macrophages by competition, relieve the inhibitory effect of CD47 that is on the tumor cells against macrophages, restore the immune function of macrophages, and exert an anti-tumor effect.

TIGIT (T cell immunoreceptor with Ig and ITIM domains, also known as VS1G9, VSTM3 or WUCAM), is a member of immunoglobulin poliovirus receptor family, CD28 receptor family, and is expressed in T cells and natural killer (NK) cell subsets. Generally, its expression levels are low, but once these cells are activated, the protein levels will be up-regulated. For example, in the tumor microenvironment, TIGIT is often expressed at high levels in tumor-infiltrating lymphocytes. TIGIT may bind to CD155 (poliovirus receptor, PVR), CD112 (PVRL2, nectin-2), and CD113 (Nectin-3), the receptors of immune cells, non-immune cells, and tumor cells, resulting in the inactivation of T cells after the binding, and the cytotoxicities of T cells and natural killer cells are inhibited.

BTLA is an important immune checkpoint molecule expressed in activated T and B lymphocytes, and has a similar structure (a single immunoglobulin variable region IgV extracellular domain) and a similar intracellular signal transduction mechanism (two ITIM domains in the cytoplasm recruit and activate SHP-1 and SHP-2 phosphatase to inhibit the function of lymphocyte) to other immune checkpoint molecules such as PD-1 and CTLA-4. HVEM (herpesvirus entry mediator) of the TNF receptor family has been identified as a ligand for BTLA and is widely expressed in human immune cells such as T cells, B cells, NK cells, myeloid cells and dendrites cells, as well as a variety of tumor cells (including non-small cell lung cancer, melanoma, colorectal cancer, and lymphoma). Under normal physiological conditions, BTLA binding to its ligand HVEM can inhibit the hyperactivation of lymphocytes in the body and prevent the immune system from self-damaging. However, tumor cells such as lung cancer, melanoma, colorectal cancer and lymphoma, which bind to BTLA expressed by tumor-specific killer lymphocytes through high expression of HVEM, may thereafter inhibit the immune function of lymphocytes. High expression of HVEM in tumors is associated with poor prognosis.

Cytokine (CK) is a small molecule polypeptide or glycoprotein synthesized and secreted by a variety of tissue cells (mainly immune cells). Cytokines can mediate interactions among cells and have a variety of biological functions, such as regulating cell growth, differentiation and maturation, maintaining function, regulating immune responses, participating in inflammatory responses, wound healing, and tumor growth and regression, etc. According to the structure and function of cytokines, cytokines can be divided into interleukin superfamily, interferon family, tumor necrosis factor superfamily, TGF-β superfamily, chemokine family, colony stimulating factor family, growth factor family and so on.

Interleukins are a class of cytokines that are generated by and act on a variety of cells. Interleukin was originally generated by leukocytes and plays a role among them, it was therefore named and is still in use today. Interleukin refers to a class of uniformly named cytokines whose molecular structure and biological functions have been generally clarified and that have important regulatory effects. It belongs to the same category of cytokines as hemocyte growth factor. The two coordinate and interact with each other to complete the functions of hematopoiesis and immune regulation. Interleukins play an important role in transmitting information, activating and regulating immune cells, mediating activation, proliferation and differentiation of T and B cells, and inflammatory responses.

IL-2 is a cytokine derived from multicellular sources (mainly generated by activated T cells) and has pleiotropic effects. It promotes the growth, proliferation, and differentiation of lymphocytes, and plays an important role in the body's immune response and antiviral infection. IL-2 can activate T cells and promote the production of cytokine; it can stimulate the proliferation of NK cells, enhance NK killing activity and produce cytokines, and induce the production of LAK cells; it can promote the proliferation of B cells and secretion of antibodies; it can activate macrophages.

IL-10, also known as cytokine synthesis inhibitory factor (CSIF), has a bidirectional immunoregulatory effect. IL-10 can exert immunosuppression through antigen-presenting cells (APCs) and exert negative regulation through T cells, having a negatively regulatory effect on the immune response in the tumor environment; in addition, IL-10 has a stimulating effect on T and B lymphocytes, and can also exert an stimulating effect in the tumor environment; the bidirectional regulatory effect of IL-10 has drawn attention since it was discovered because it not only affects the immune system, but also affects many pathophysiological processes, including angiogenesis, tumor formation and infection, by regulating growth factors and cytokines, and can also establish a role in peripheral tolerance by inducing regulatory T cells; IL-10 plays an important role in Crohn's disease, rheumatoid arthritis and psoriasis, HCV infection, HIV infection, etc. In the natural state, IL-10 activates downstream signaling pathways by forming a homodimer and binding to its receptor to form a complex, and exerts its physiological functions. In 2000, Josephson *et al.* studied and designed IL-10 mutants, which can bind to IL-10 receptors and activate downstream signaling pathways in the state of monomers (Josephson, K. Design and analysis of an engineered human interleukin-10 monomer.[J]. Journal of Biological Chemistry, 2000, 275(18): 13552-7.).

Interferon is a group of active proteins with various functions, which is a cytokine mainly generated by monocytes and lymphocytes, and is divided into three categories, including type I, type II, and type III. Type I interferons are mainly IFN-α and IFN-β, which are secreted by innate immune cells, type II interferons are IFN-γ, which are mainly secreted by activated T cells, and type III interferons are several types of IFN-λ. Interferon has various biological activities, such as broad-spectrum anti virus, influences on cell growth and differentiation, regulation of immune function, etc. The specific functions are as follows:
(1) Antiviral effect: type I interferon is the main antiviral defense and regulator in the immune system. During the period of early viral infection of the body, type I interferon can control the growth and proliferation of the virus. On the one hand, it directly activates immune cells, and on the other hand, it can indirectly inhibit the replication process of the virus.
(2) Antibacterial effect: interferon can reduce the iron supply of bacteria by downregulating transferrin receptors or directly inhibit intracellular bacteria by inducing the production of endogenous NO, and can also increase the bacteriolysis of the phagosome-lysosome of mononuclear macrophages, and eventually achieve the effect of eliminating bacteria through the above ways.
(3) Antiparasitic effect: interferon can activate macrophages (Mϕ), activated Mϕ can express high levels of inducible nitric oxide synthase (iNOS) which catalyzes the L-arginine to produce NO. NO has an inhibitory and killing effect on inoculated pathogens. As it is reported, IFN-γ can activate Mϕ to produce NO, and at the same time promote NO synthesis in a dose-dependent manner, the higher the dose, the more obvious the effect. Daubener *et al.* (2001) found that stimulating human brain microvascular endothelial cells (HBMEC) with IFN-γ can induce their resistance to toxoplasmosis. IFN-γ stimulated HBMECs can inhibit the growth of toxoplasma gondii and increase the appearance of TNF-α, which is related to the activity of IDO. In addition, the addition of excessive tryptophan during the culture of HBMEC can completely inhibit IPN-γ-TNF-α-mediated resistance to toxoplasmosis, suggesting that IDO can mediate its protection, and it is reported that IFN-γ works depending on the expression of IDO.
(4) Participating in immune regulation: the main part participating in immune regulation is IFN-γ, also known as immunoregulatory interferon. Immunoregulatory interferon can enhance the expression of the Fc receptor of IgG, which is conducive to the phagocytosis of antigens by macrophages, the killing of target cells by K and NK cells, and the activation of T and B lymphocytes, and thereby enhancing the immune response ability of the body. IFN-γ can increase the expression of class II MHC molecules on the surface of macrophages and enhance their antigen presentation ability. In addition, IFN-γ can also promote the phagocytosis of immune complexes, antibody-coated pathogens and tumor cells by macrophages through enhancing the expression of Fc receptors on the surface of macrophages. At the same time, IFN-γ can also stimulate neutrophils, enhance their phagocytic ability, activate NK cells, and enhance their cytotoxic effects to participate in immune regulation.
(5) Anti-tumor effect: IFN-α and IFN-β have a wide range of anti-tumor effects. IFN-γ has various regulatory effects on the immune response of the body, it can activate effector cells, increase the activities of natural killer cells, macrophages and tumor-infiltrating lymphocytes, promote the circulation of monocytes, enhance the expression of antigens and antibodies on the surface of immune cells, stimulate the production of cytokines such as IL-2, tumor necrosis factor, IFN-α, etc., inhibit tumor cell division, and induce genes to fully become antiviral proteins, etc.

Of the tumor necrosis factor superfamily, tumor necrosis factor (TNF) is a cytokine found in serum capable of killing cancer cells in mice. Members mainly comprise TNF-α, TNF-β, lymphotoxin-β, CD40L, FasL, CD30L, 4-1BBL, CD27L and OX40L, TNF-related apoptosis-inducing ligand (TRAIL), receptor activator of LIGHT (RANKL), TNF-related weak inducer of apoptosis (TWEAK), proliferation-inducing ligand (APRIL), B cell activating factor (BAFF), vascular endothelial growth inhibitor (VEGI), ectodysplasin A (EDA-A1, EDA-A2) and glucocorticoid-induced tumor necrosis factor receptor-related ligand (GITRL). Members of tumor necrosis factor TNF superfamily specifically recognize dozens of receptors and constitute a ligand-receptor interaction system. TNF receptors (TNFRs) are mainly transmembrane proteins, involved in some physiological processes, such as host defense, inflammation, apoptosis, autoimmunity, and immune, development and organogenesis of ectodermal and nervous system. TNF causes tumor cell necrosis (cell swelling, organelle destruction, cell lysis) and apoptosis (cell shrinkage, aggregate formation, DNA fragmentation) and plays a key role in various immune and inflammatory processes.

Colony stimulating factor (CSF) is a cytokine that stimulates the differentiation and maturation of immature bone marrow cells and stimulates colony formation *in vitro.* Different cell colonies are formed in semi-solid medium according to the different hematopoietic cell lines or cells at different differentiation stages stimulated by cytokines, and are named as granulocyte CSF (G-CSF), macrophage CSF (M-CSF), granulocyte and macrophage CSF (GM-CSF), multiple colony stimulating factor (multi-CSF, also known as IL-3), stem cell factor (SCF), erythropoietin (EPO), respectively. They play a role in promoting proliferation and differentiation of hematopoietic stem cells at different developmental stages, and are essential stimulators for hematopoiesis. Broadly speaking, all cytokines that stimulate hematopoiesis can be collectively referred to as CSF. For example, leukemia inhibitory factor (LIF), which can stimulate embryonic stem cells, and thrombopoietin, which stimulates platelets, have colony-stimulating activity. In addition, CSF also acts on a variety of mature cells, promoting their functions with heterogeneous effects.

Chemokines are a class of small cytokines or signaling proteins secreted by cells. They are named chemokines due to their ability to induce directed chemotaxis of nearby responding cells. Common structural features of chemokine proteins include small molecular weight (about 8-10 kDa) and four conserved cysteine residues to ensure their tertiary structure. Chemokines are divided into four major subfamilies: CXC, CC, CX3C and XC. All of these proteins exert their biological effects by interacting with G protein-linked transmembrane receptors (known as chemokine receptors). These proteins work by binding to chemokine receptors, which are G protein-coupled transmembrane receptors selectively expressed on the surface of target cells.

The main function of chemokines is to manage the migration (homing) of leukocytes to their respective locations during inflammation and homeostasis. Basic homing effect: basal homeostatic chemokine are produced in thymus and lymphoid tissue. The chemokines CCL19 and CCL21 (expressed in lymph node and lymphatic endothelial cells) and their receptor CCR7 (expressed in cells destined to home to these organs) are the best illustration of their homeostatic function in homing. Utilizing these ligands allows antigen-presenting cells (APCs) to metastasize to lymph nodes during an adaptive immune response. Other homeostatic chemokine receptors including CCR9, CCR10, and CXCR5, are important for tissue-specific leukocyte homing as part of the cellular address. CCR9 supports leukocyte's migration into the intestine, CCR10 supports skin migration, and CXCR5 supports B cell's migration into lymph node follicles. CXCL12 (SDF-1) generated in the bone marrow promotes the proliferation of B progenitors in the bone marrow microenvironment. Inflammatory homing effect: inflammatory chemokines are produced in high concentrations during infection or injury, and determine the migration of inflammatory leukocytes to damaged areas. Typical inflammatory chemokines comprise CCL2, CCL3, CCL5, CXCL1, CXCL2 and CXCL8.

Some chemokines are considered pro-inflammatory cytokines that may induce cells of the immune system to enter the infection site during the process of immune response. Whereas some chemokines are considered to maintain self-regulation of the body and control cell migration during the process of normal tissue maintenance or development.

Chemokines can be divided into the following categories according to the type of cells they perform chemotaxis in:
Monocyte/macrophage Chemokines: key chemokines that attract monocytes/macrophages to the site of inflammation comprise CCL2, CCL3, CCL5, CCL7, CCL8, CCL13, CCL17, and CCL22.

T lymphocyte chemokines: four key chemokines involved in the recruitment of T lymphocytes to the site of inflammation are CCL2, CCL1, CCL22, and CCL17. In addition, expression of CXCR3 is induced after T cell activation, and the activated T cells are attracted by the site of inflammation, where they secrete IPN-γ-induced chemokines CXCL9, CXCL 10, and CXCL11.

Mast cell chemokines: a variety of chemokine receptors are expressed on the surface: CCR1, CCR2, CCR3, CCR4, CCR5, CXCR2, CXCR4. Ligands of these receptors, CCL2 and CCL5, play important roles in recruitment and activation of lung mast cells. There is also evidence that CXCL8 may inhibit mast cells.

Eosinophil chemokines: migration of eosinophils to various tissues involves several chemokines of the CC family: CCL11, CCL24, CCL26, CCL5, CCL7, CCL13, and CCL3. The chemokines CCL11 (eotaxin) and CCL5 (rantes) work through a specific receptor CCR3 that is on the surface of eosinophils, while eosinophils play an important role in the initial recruitment to the lesion.

Neutrophil chemokines: mainly regulated by CXC chemokines. For example, CXCL8 (IL-8) is a chemoattractant for neutrophils and activates their metabolism and degranulation.

The TGF-β superfamily is a group of cytokines that regulate cell growth and differentiation. In addition to TGF-β, this family also comprises activins, inhibins, Mullerian inhibitor substance (MIS) and bone morpho-genetic proteins (BMPs). TGF-β plays a role in inflammation, tissue repair and embryonic development, and has important regulatory effects on cell growth, differentiation and immune function. The specific functions are as follows:
(1) Inhibition of the proliferation of immunocompetent cells: i) inhibiting the colony formation of mouse hematopoietic precursor cells and LTBMC induced by IL-3, GM-CSF and M-CSF, and reducing the reactivity of megakaryocytes to IL-3T and CSF. ii) Inhibiting the proliferation of thymocytes induced by ConA or co-induced by ConA and IL-2, IL-6. iii) Inhibiting the proliferation of T cell stimulated by mitogen and allogeneic antigen or the growth of IL-2-dependent T cell. iv) Inhibiting the proliferation of IL-2-dependent B cell after the stimulation of SAC.
(2) Regulation of cell phenotype: i) inhibiting the expression of T cells IL-2R, TfR and TLiSA1 activation antigen induced by IL-2, with no influence on CD3 expression observed. ii) Inhibiting the expression of class II MHC antigen in melanoma cells induced by IFN-γ.
(3) Inhibition of the differentiation of lymphocytes: i) inhibiting IL-2 and BCDF-dependent B cells to secrete IgM, and promoting the secreted Ig type of B cells to convert into IgA and IgE. ii) Inhibiting the functions of CTL, NK, and LAK in mixed lymphocyte culture (MLC), which can be reversed by TNF-α (mouse MIC) or IL-2 (human MLC). iii) Inhibiting the activity of NK in PBMC. iv) Inhibiting the activity of MHC non-restricted killer cells in mouse thymus synergistically induced by ConA and IL-2, IL-6.
(4) Inhibition of the production of cytokine: such as inhibiting the production of IFN-γ and TNF-α in PBMC.
(5) Other regulatory effects: i) promoting the growth of fibroblasts, osteoblasts and Schwann cells. TGF-β1 and TGF-β2 promote the production of IL-6 in human fibroblasts, and its mechanism may be through the regulation of IL-6 gene transcription, ii) Inhibiting the growth of epithelial cells, osteoclasts, and endothelial cells, and the formation of fat, cardiac muscle and skeletal muscle. TGF-β can antagonize some biological functions of EGF. iii) Promoting the expression of extracellular matrix (ECM) such as collagen and fibronectin, and inhibiting the degradation of ECM, which plays an important role in the processes of cell morphogenesis, proliferation and differentiation and is beneficial to embryonic development and cell repair. *In vivo* experiments in animals have shown that local injection of TGF-β can promote wound healing and the formation of typical granulation tissue. iv) A chemoattractant for monocytes and fibroblasts, with no colloidal particles and oxides produced yet. v) Inhibiting the adhesion between lymphocytes and endothelial cells, vi) Promoting the release of histamine from basophils.
(6) Expression of TGF-β1 and proto-oncogene: TGF-β1 can induce the expression of c-sis, but inhibit the expression of c-myc. Such induction or inhibition is related to the type of cells and the different functions of TGF-β. For example, the expression of c-sis gene in fibroblasts induced by TGF-β is related to the promotion of their growth in soft agar; while the inhibition of the growth of epithelial keratinocytes is related to the inhibition of gene expression of c-myc. TGF-β1, TGF-β2, and TGF-β3 are very similar in most biological roles, but can vary in some roles, such as the growth inhibitory effect of TGF-02 on vascular endothelial cells and hematopoietic progenitor cells, which is only 1% of TGF-β1 and TGF-β3.

TGF-β has potential applications in terms of treating wound healing, promoting cartilage and bone repair, and treating autoimmune diseases and transplant rejection through immunosuppression.

Growth factors are a class of cytokines secreted by a variety of cells, acting on specific target cells and regulating cell division, matrix synthesis and tissue differentiation. There are many kinds of growth factors, such as platelet growth factor (platelet-derived growth factor, PDGF; osteosarcoma-derived growth factor, ODGF), epidermal growth factor (EGF), fibroblast growth factor (αFGF, βFGF), insulin-like growth factor (IGF-I, IGF-II), nerve growth factor (NGF), etc.

However, immunoregulation is usually systemic, and immune activation is often accompanied by immune system hyperactivation, resulting in the following immune-related toxic reactions:
skin: manifested as rash/maculopapular rash, pruritus, bullous dermatitis syndrome, etc.;
endocrine: manifested as hypothyroidism, hyperthyroidism, primary adrenal insufficiency, hyperglycemia, etc.;
liver: mainly manifested as elevated transaminases;
gastrointestinal: mainly manifested as diarrhea/colitis;
lungs: immune-related pneumonia;
there are other relatively rare adverse reactions, including neurotoxicity, hematological toxicity, renal toxicity, cardiotoxicity, ocular toxicity, etc.

Therapies targeting immune checkpoints or their ligands, cytokines or their receptors have shown therapeutic effects on tumors, autoimmune diseases, sepsis, graft-versus-host disease (GVHD) and other diseases, but underlying toxicities associated with the drug itself limit the dose of the drug and reduce its efficacy. In addition, immunosuppression is often accompanied by an excessively low immune system, which easily leads to bacterial, viral, and fungal infections, and results in recurrent inflammation and tumorigenesis.

Therefore, a single targeted therapy, such as various cytokines, monoclonal antibodies, ligand/receptor Fc fusion proteins, etc., is often insufficient to achieve the best therapeutic window and therapeutic effect. The concept of therapy with the synergistic effect of two or more targets emerges. In this case, bispecific antibodies/fusion proteins (bi-clonal antibody molecules) or multi-specific antibodies/fusion proteins (polyclonal antibody molecules) targeting two or more targets may provide new therapeutic applications that are difficult to achieve with monoclonal antibodies. These include the design thinking and clinical application of bi/multi-specific antibodies/fusion proteins (e.g., Blinatumomab) targeting target antigen on the surface of target cells (e.g., CD19, HER2, etc.) and targeting immunoregulatory targets (e.g., CD3, PD-1, CD47, etc.) respectively, or carrying immunoregulatory agents (e.g., IL-2, 4-1BB, SIRPα, CD80, etc.).

Although the common bi-clonal antibody molecule/bispecific fusion protein with symmetric structure has a certain targeted aggregation effect, which enables the drug to distribute in the target organ and accumulate to a certain extent, and makes the pharmaceutical development process, especially the purification process relatively controllable, there are still some limitations in its structure, especially the difference of drug concentration between target organs and non-target organs is not significant, and if non-target organ administration such as subcutaneous or intravenous administration is adopted, the drug concentration in the blood is still the main problem that results in immunotoxicity and restricts its safe dosage. Therefore, the combination of the affinity of the antigen-binding fragment/fusion protein targeting the target antigen and the antigen-binding fragment/fusion protein targeting the immune checkpoint/cytokine receptor needs to be strictly controlled, so that when the part of bispecific molecule targeting the target antigen has not achieved the optimal concentration-enriched effect in the target cell, the immune-related toxic side effects caused by the part of bispecific molecule targeting immunoregulation can be prevented.

In addition to the symmetrical structure, there are also bispecific antibodies/fusion proteins that rely on the affinity difference between the left and right arms to further improve the enrichment of drugs on the surface of targeted antigen-positive cells through asymmetrical structures, thereby further reducing the immune-related toxic side effects caused by the part targeting immunoregulation. However, in addition to the unfavorable factors of asymmetric structures such as increased difficulty in pharmaceutical process, reduced yield, and increased production costs, the monomer or polymer impurities of immunoregulatory arms have become an important source of risk causing immune-related adverse events. Taking TTI-621 (SIRPα-Fc fusion protein developed by Trillium Therapeutics Inc.) as an example, according to the published clinical research results of TTI-621, at an extremely low dose (0.2 mg/kg), it can cause more than 20% of patients to have serious side effects of grade three or above thrombocytopenia (due to TTI-621 binding to the highly expressed CD47 on platelets, which results in platelets being cleared by immune cells). As the bi-clonal antibody structure shown in Figure 3 of the specification in CN108864290A, the finished product will potentially contain impurities of the right arm monomer/dimer structure (i.e., TTI-621 analog) during the preparation process. Based on the dosage of 5 mg/kg-20 mg/kg as the dosage of antibody drugs for conventional tumor-targeted therapies, it can reach the dose concentration of TTI-621 with severe toxic side effects at an extremely low impurity concentration (1-4%). Therefore, this structure's control on the impurity content in the finished product is extremely strict and the production costs will significantly increase.

In addition, some immunoregulatory fusion proteins, such as interferon, generally have poor freeze-thaw stability due to their characteristics. For example, the aggregations gradually increase after the recombinant human albumin interferon-α2b fusion protein is recurrently freeze-thawed. After 4 times of freeze-thaw, the content of the aggregations reaches 17.91%, and thus the recombinant human albumin interferon-α2b fusion protein should not be freeze-thawed (Yi Xia et al., The Stability Study of Recombinant Human Albumin Interferon α-2b Fusion Protein, Journal of Biology, 2008, 25(006):38-40), so as to cause more restrictions on production, transportation and use.

Therefore, it is urgent to invent a bi-clonal antibody or bispecific structure that can simultaneously target the antigen of interest and exert an immunoregulatory effect. Such structure not only enables the target cells to be enriched at high concentrations and exerts an immunoregulatory effect, but also avoids exerting the immunoregulatory function solely relying on the immunoregulatory part in the absence of the antigen of interest, even though the concentration is relatively high in the blood, so as to achieve more precise immunoregulations of target organs, tissues and cells, reduce the risk of immune-related toxic side effects, and at the same time improve the stability of the product (such as freeze-thaw stability).

### CONTENT OF THE PRESENT INVENTION

The first technical problem to be solved by the present disclosure is to provide a bispecific recombinant protein with directional regulation which has strong targeting to target cells of interest containing the antigen of interest, and at the same time does not bind or weakly binds to non-target cells of interest not containing the antigen of interest. It significantly improves the targeting of the second functional binding fragment of the recombinant protein to target cells of interest, directionally regulates the immune effect, and significantly reduces toxic side effects caused by the potentially risky impurities containing the second functional binding fragment and generated during the preparation process targeting to the organs and tissues of the non-target cells of interest.

The second technical problem to be solved by the present disclosure is to provide a use of the bispecific recombinant protein with directional regulation in the manufacture of a medicament.

The third technical problem to be solved by the present disclosure is to provide a bispecific recombinant protein that can improve product stability (such as freeze-thaw stability), addressing the product instability caused by the product characteristics of some immunoregulatory fusion proteins (such as interferon, etc.) and the consequent restrictions on production, transportation, and use.

In one aspect, the present disclosure provides a bispecific recombinant protein, the bispecific recombinant protein comprises a first functional binding fragment, a second functional binding fragment, and an Fc region; the first functional binding fragment targeting an antigen of interest of the bispecific recombinant protein comprises an antigen-binding fragment, wherein a C-terminus of a CL domain or a C-terminus of a CH1 domain in the antigen-binding fragment is directly connected to or is connected via a linker to the second functional binding fragment having a function of immunoregulation and/or a function of metabolic regulation and/or a function of endocrine regulation.

Preferably, the antigen-binding fragment is directly connected to or is connected via a linker to a N-terminus of the second functional binding fragment, and, a C-terminus of the second functional binding fragment is directly connected to or is connected via a linker to a N-terminus of the Fc region.

In one embodiment, the second functional binding fragment having a function of immunoregulation targets an immune checkpoint, an immune checkpoint ligand, or a cytokine receptor. Optionally, the second functional binding fragment having a function of immunoregulation targets PD-1 or a ligand thereof, CD47 or a ligand thereof, CD24 or a ligand thereof, a interferon receptor (such as type I or type II interferon receptor), a interleukin receptor.

In one embodiment, the second functional binding fragment having a function of metabolic regulation targets a metabolic regulator, a metabolic regulator receptor. Optionally, the second functional binding fragment having a function of metabolic regulation targets an insulin receptor, a fibroblast growth factor receptor.

In one embodiment, the second functional binding fragment having a function of endocrine regulation targets an endocrine regulator, an endocrine regulator receptor. Optionally, the second functional binding fragment having a function of endocrine regulation targets a hormone receptor.

In one embodiment, a variable region (V region) and a constant region (C region) in the antigen-binding fragment are directly connected or are connected via a linker; or the antigen-binding fragment and the Fc region are directly connected or are connected via a linker; or both of methods mentioned above are used simultaneously to connect.

In one embodiment, the linker sequence comprises (GGGGS)n, (GGGS)n, (GGS)n, (G)n, (GS)n, (EAAAK)n, or (XP)n, n is a natural number. Preferably, n is a natural number from 0 to 5.

In one embodiment, the linker sequence is (GGGGS)n, n=0, 1, 2, 3, 4, 5.

In one embodiment, the second functional binding fragment binds to a cytokine receptor, an immune checkpoint or an immune checkpoint ligand. Preferably, the second functional binding fragment is the cytokine, the immune checkpoint ligand or a binding protein for the immune checkpoint ligand, or a functional fragment thereof or a mutant thereof; optionally, an extracellular functional fragment of human SIRP family, a functional fragment of human interferon family, a functional fragment of tumor necrosis factor superfamily, a functional fragment of TGF-β superfamily, a functional fragment of interleukins, a functional fragment of chemokine family, a functional fragment of colony stimulating factor family, a functional fragment of growth factors, or a mutant thereof.

In one embodiment, the second functional binding fragment is an extracellular D1 domain of human SIRPα or a mutant thereof. Preferably, the mutant is a low-affinity mutant, i.e., the binding affinity of this mutant to human CD47 protein is not higher than the binding affinity of the wild-type protein to human CD47 protein.

In one embodiment, the second functional binding fragment is a human interferon γ (IFN-γ) or a human interferon α (IFN-α) or a human interferon β (IFN-β), a truncated variant thereof, or a mutant thereof.

In one embodiment, the second functional binding fragment is an interleukin, a truncated variant thereof, or a mutant thereof.

In one embodiment, the interleukin is an immunoregulatory factor or chemokine selected from any one of the following: IL-1 family, IL-2 family, IL-3 family, IL-6 family, IL-8 family, IL-10 family, IL-12 family, and IL-17 family. Preferably, the second functional binding fragment is a human IL-10 monomer mutant (amino acid sequence as shown in SEQ ID NO: 52), human IL-12A or a mutant thereof (amino acid sequence as shown in SEQ ID NO: 51), a fusion protein or a mutant thereof consisting of human IL-15 (amino acid sequence as shown in SEQ ID NO: 53) and IL-15RαSUSHI (amino acid sequence as shown in SEQ ID NO: 54) by direct connection or via a linker.

In one embodiment, the first functional binding fragment targets any one or more of the following targets: 5T4, AGS-16, ALK1, ANG-2, B7-H3, B7-H4, c-fms, c-Met, CA6, CD123, CD19, CD20, CD22, CD24, EpCAM, CD30, CD32b, CD37, CD38, CD40, CD52, CD70, CD71, CD74, CD79b, CD80, CD83, CD86, CD98, CD206, CEA, CEACAM5, CLDN18.2, CLDN6, CS1, CCR5, CXCR4, DLL-4, EGFR, EGFRvIII, EGP-1, ENPP3, EphA3, ETBR, FGFR2, FN, FR-α, GCC, GD2, GPC-3, GPNMB, HER2, HER3, HLA-DR, ICAM-1, IGF-1R, IL-3R, LIV-1, MSLN, MUC16, MUC1, NaPi2b, Nectin-4, Notch 2, Notch 1, PD-1, PD-L1, PD-L2, PDGFR-α, PS, PSMA, SLTRK6, STEAP1, TEM1, TIGIT, VEGFR, CD25, CD27L, DKK-1, CSF-1R, MSB0010718C, BCMA, CD138, TROP2, Siglec15, CD155, and AFP.

Preferably, when the second functional binding fragment comprises an extracellular D1 domain of human SIRPα or a mutant thereof, the first functional binding fragment targets a tumor cell or an immune cell.

When the second functional binding fragment is a human interferon α (IFN-α) or a human interferon β (IFN-β) or a human interferon γ (IFN-γ), a truncated variant thereof, or a mutant thereof, the first functional binding fragment targets a tumor cell or an immune cell.

When the second functional binding fragment is an interleukin, a truncated variant thereof, or a mutant thereof, the first functional binding fragment targets a tumor cell or an immune cell.

In one embodiment, the bispecific recombinant protein consists of a chain A and a chain B, the chain A binds to the chain B by intermolecular force, by covalent bond, such as interchain disulfide bond, or by salt bond, or by a combination of two or three of binding methods mentioned above. The chain A is a protein consisting of VH, CL/CH1, CH2, CH3 domains in order, or a protein consisting of VL, CL/CH1, CH2, CH3 domains in order, the domains may be connected directly or connected via a linker (Linker); the chain B is a protein consisting of VL, CL/CH1, a second functional binding fragment, CH2, CH3 domains in order, or a protein consisting of VH, CL/CH1, a second functional binding fragment, CH2, CH3 domains in order, the domains may be connected directly or connected via a linker (Linker); for example, when the "chain A" is VH-CH1-CH2-CH3, then the "chain B" is VL-CL-the second functional binding fragment-CH2-CH3; when the "chain A" is VL-CL-CH2-CH3, then the "chain B" is VH-CH1-the second functional binding fragment-CH2-CH3, when the "chain A" is VH-CL-CH2-CH3, then the "chain B" is VL-CH1-the second functional binding fragment-CH2-CH3; when the "chain A" is VL-CL-CH2-CH3, then the "chain B" is VH-CL-the second functional binding fragment-CH2-CH3. The N-terminus of the CH2 further contains a hinge region.

In one embodiment, the Fc region of the bispecific recombinant protein comprises an Fc region native sequence or an Fc region non-native sequence; more preferably, the Fc region is a human Fc region; further more preferably, the Fc region of the chain A binds to the Fc region of the chain B by knobs-into-holes.

In one embodiment, the Fc region is an Fc region of human IgG; preferably, the Fc region is an Fc region of human IgG1 or IgG4.

In one embodiment, the first functional binding fragment is an antigen-binding fragment, optionally a human-mouse chimeric antigen-binding fragment, a humanized antigen-binding fragment, or a fully human antigen-binding fragment; the first functional binding fragment is selected from any one or more of antigen-binding fragments of the following target antibodies: 5T4, AGS-16, ALK1, ANG-2, B7-H3, B7-H4, c-fms, c-Met, CA6, CD123, CD19, CD20, CD22, CD24, EpCAM, CD30, CD32b, CD37, CD38, CD40, CD52, CD70, CD71, CD74, CD79b, CD80, CD83, CD86, CD98, CD206, CEA, CEACAM5, CLDN18.2, CLDN6, CS1, CCR5, CXCR4, DLL-4, EGFR, EGFRvIII, EGP-1, ENPP3, EphA3, ETBR, FGFR2, FN, FR-α, GCC, GD2, GPC-3, GPNMB, HER2, HER3, HLA-DR, ICAM-1, IGF-1R, IL-3R, LIV-1, MSLN, MUC16, MUC1, NaPi2b, Nectin-4, Notch 2, Notch 1, PD-1, PD-L1, PD-L2, PDGFR-α, PS, PSMA, SLTRK6, STEAP1, TEM1, TIGIT, VEGFR, CD25, CD27L, DKK-1 , CSF-1R, MSB0010718C, BCMA, CD138, TROP2, Siglec15, CD155, andAFP; preferably, the first functional binding fragment is a humanized or a fully human antigen-binding fragment.

In one embodiment, when the first functional binding fragment targets CD20, EGFR, EGFRvIII, PD-L1, PD-L2, HER2, HER3, CD138, CD44, CD24, EpCAM, CLDN18.2, CD38, BCMA, MUCl, or TROP2, the second functional binding fragment comprises an extracellular D1 domain of SIRPα or a mutant thereof. Preferably, the second functional binding fragment is as shown in SEQ ID NO: 50.

In one embodiment, when the first functional binding fragment targets Siglec15, PD-L1, PD-L2, CD71, CD80, CD86, CD206, CCR5, the second functional binding fragment comprises an IFN-α or IFN-β or IFN-γ or IL-10 monomer mutant or a fusion protein formed by IL-12A or IL-15 and IL-15RαSUSHI or a truncated variant of the above-mentioned cytokine or a mutant retaining a cytokine function. More preferably, the second functional binding fragment comprises a fusion protein formed by cytokines as shown in SEQ ID NO: 51, or SEQ ID NO: 52, or SEQ ID NO: 53, and SEQ ID NO: 55.

In one embodiment, the first functional binding fragment is an antigen-binding fragment of anti-CD20 antibody Ofatumumab, or anti-EGFR antibody Panitumumab, or anti-EpCAM antibody Catumaxomab, or anti-CD24 antibody SWA11, the second functional binding fragment is an extracellular D1 domain of human SIRPα (as shown in SEQ ID NO: 50).

In one embodiment, the first functional binding fragment comprises an antigen-binding fragment of anti-AFP antibody tacatuzumab, the second functional binding fragment is human IFN-α 2b or human IFNβ (SEQ ID NO: 55).

In one embodiment, the chain A and chain B of the bispecific recombinant protein is bound by IgG Fc, preferably, by knobs-into-holes. For example, the knobs-into-holes is a convex "knobs" type formed by T366W mutation, and a concave "holes" type formed by one amino acid mutation (T407V) or three amino acid mutations (T366S, L368A and Y407V). According to the Kabat numbering scheme [Eu numbering scheme of Kabat et al. (1991)], the mutation is shown as the original amino acid residue, the mutation site and the substituted amino acid residue, in order from left to right respectively, for example, in T366W, T is the original amino acid residue, 366 is the mutation site, and W is the T-substituted amino acid residue.

In some embodiments, the first functional binding fragment targets CD20, EpCAM, CD24, or EGFR, the second functional binding fragment comprises an extracellular D1 domain of human SIRPα and a mutant thereof; preferably, the amino acid sequence of the second functional binding fragment is as shown in SEQ ID NO: 50; more preferably, the amino acid sequence of the chain A is as shown in SEQ ID NO: 1, the amino acid sequence of the chain B is as shown in SEQ ID NO: 2 or 3; the amino acid sequence of the chain A is as shown in SEQ ID NO: 61, the amino acid sequence of the chain B is as shown in SEQ ID NO: 62; the amino acid sequence of the chain A is as shown in SEQ ID NO: 27, the amino acid of the chain B is as shown in SEQ ID NO: 28; the amino acid sequence of the chain A is as shown in SEQ ID NO: 29, the amino acid sequence of the chain B is as shown in SEQ ID NO: 30; the amino acid sequence of the chain A is as shown in SEQ ID NO: 56, the amino acid sequence of the chain B is as shown in SEQ ID NO: 57.

In some embodiments, the first functional binding fragment targets TIGIT, CD80, or PD-1, the second functional binding fragment comprises IL-12A, an IL-10 monomer mutant, a complex formed by IL15 and IL-15RαSUSHI, their amino acid sequences are as shown in SEQ ID NO: 51, 52, 53-54 respectively; preferably, the amino acid sequence of the chain A is as shown in SEQ ID NO: 35, the amino acid sequence of the chain B is as shown in SEQ ID NO: 36; the amino acid sequence of the chain A is as shown in SEQ ID NO: 37, the amino acid sequence of the chain B is as shown in SEQ ID NO: 38; the amino acid sequence of the chain A is as shown in SEQ ID NO: 39, the amino acid sequence of the chain B is as shown in SEQ ID NO: 40 or 41.

In some embodiments, the first functional binding fragment targets CD38 or AFP, the second functional binding fragment comprises an extracellular D1 domain of human SIRPα and a mutant thereof, or IFN-β or a mutant thereof; preferably, the amino acid sequence of the second functional binding fragment is as shown in SEQ ID NO: 50 or 55; more preferably, the amino acid sequence of the chain A is as shown in SEQ ID NO: 31, the amino acid sequence of the chain B is as shown in SEQ ID NO: 32; the amino acid sequence of the chain A is as shown in SEQ ID NO: 33, the amino acid sequence of the chain B is as shown in SEQ ID NO: 34; or, the amino acid sequence of the chain A is as shown in SEQ ID NO: 58, the amino acid sequence of the chain B is as shown in SEQ ID NO: 60.

In another aspect, the present disclosure provides a nucleic acid molecule encoding the bispecific recombinant protein; wherein, the nucleic acid molecule encoding the first functional binding fragment and the nucleic acid encoding the second functional binding fragment are in a same DNA strand, or the nucleic acid molecule encoding the first functional binding fragment and the nucleic acid encoding the second functional binding fragment are in different DNA strands.

Preferably, the nucleic acid molecule encoding the Fc region and the nucleic acid encoding the first functional binding fragment or the second functional binding fragment are in a same DNA strand, the nucleic acid molecule encoding the Fc region and the nucleic acid encoding the first functional binding fragment or the second functional binding fragment are in different DNA strands.

In another aspect, the present disclosure provides an expression vector comprising the nucleic acid molecule.

In another aspect, the present disclosure provides a host cell transformed with the expression vector.

In another aspect, the present disclosure further provides a method for preparing the bispecific recombinant protein, the expression vector is used to transform into the host cell, the host cell is cultured under a condition suitable for expression, and then the bispecific recombinant protein is obtained by expression.

In another aspect, the present disclosure provides a use of the bispecific recombinant protein in the manufacture of a medicament for the treatment of tumors, autoimmune diseases, infectious diseases, sepsis, graft-versus-host diseases, metabolic disorders, endocrine disorders.

In one embodiment, the tumor is a solid tumor or a hematological tumor; preferably, the solid tumor is selected from a group consisting of breast cancer, colorectal cancer, lung cancer, pancreatic cancer, esophagus cancer, endometrial cancer, ovarian cancer, stomach cancer, prostate cancer, kidney cancer, cervical cancer, thyroid cancer, uterine cancer, bladder cancer, neuroendocrine cancer, head and neck cancer, liver cancer, nasopharyngeal cancer, testicular cancer, small cell lung cancer, non-small cell lung cancer, melanoma, basal cell skin cancer, squamous cell skin cancer, dermatofibrosarcoma protuberans, Merkel cell carcinoma, glioblastoma, glioma, sarcoma, mesothelioma, or myelodysplastic syndrome, and the like; the hematological tumor is selected from myeloma, lymphoma or leukemia; preferably, the autoimmune disease is selected from any one of the following: Hashimoto's thyroiditis, type 1 diabetes, systemic lupus erythematosus, rheumatoid arthritis, sjogren syndrome, and the like; preferably, the infectious disease is selected from any one of the following: viral infections, bacterial infections, fungal infections, other pathogenic infections, and the like.

In another aspect, the present disclosure provides a medicament or pharmaceutical composition, which comprises the bispecific recombinant protein of the present disclosure and an optional adjuvant, excipient or a pharmaceutically acceptable carrier. The pharmaceutical composition may contain a pharmaceutically acceptable carrier. The composition may exist in any form of pharmaceutical preparation, including but not limited to injection, powder, freeze-dried powder, etc. The pharmaceutical composition in the form of the pharmaceutical preparation may be prepared according to the conventional technology of pharmaceutics, including the fusion of the active ingredient of the medicament, the bispecific recombinant protein or fusion protein of the present disclosure with the pharmaceutical carrier, and the required dosage form can be prepared according to the conventional technology of pharmaceutics.

In one embodiment, the present disclosure further provides a pharmaceutical composition, comprising an expression vector of the nucleic acid molecule encoding the bispecific recombinant protein of the present disclosure, and optionally a pharmaceutically acceptable carrier.

In another aspect, the present disclosure further provides a method for the treatment of tumor, comprising administrating a therapeutically effective amount of the pharmaceutical composition of the present disclosure to a patient or a subject. The tumor expresses an additional target molecule, the targets include but are not limited to 5T4, AGS-16, ALK1, ANG-2, B7-H3, B7-H4, c-fms, c-Met, CA6, CD123, CD19, CD20, CD22, CD24, EpCAM, CD30, CD32b, CD37, CD38, CD40, CD 52, CD70, CD71, CD74, CD79b, CD80, CD83, CD86, CD98, CD206, CEA, CEACAM5, CLDN18.2, CLDN6, CS1, CCR5, CXCR4, DL-4, EGFR, EGFRvIII, EGP-1, ENPP3, EphA3, ETBR, FGFR2, FN, FR-α, GCC, GD2, GPC-3, GPNMB, HER2, HER3, HLA-DR, ICAM-1, IGF-1R, IL-3R, LIV-1, MSLN, MUC16, MUC1, NaPi2b, Nectin-4, Notch 2 Notch 1, PD-L1, PD-L2, PD-1, PDGFR-α, PS, PSMA, SLTRK6, STEAP1, TEM1, TIGIT, VEGFR, CD25, CD27L, DKK-1, CSF-1R, MSB0010718C, BCMA, CD138, TROP2, Siglec15, CD155 or AFP.

In another aspect, the present disclosure further provides an *in vivo* gene therapy, comprising introducing a therapeutically effective amount of the nucleic acid molecule or derivative thereof encoding the recombinant protein or fusion protein of the present disclosure to a patient or a subject.

As used herein, the term "recombinant protein" refers to an artificially designed/constructed protein, rather than a naturally occurring protein. The "recombinant" in the "recombinant protein" of the present disclosure does not represent its production mode, it is only used to indicate that the "recombinant protein" does not exist in nature. The recombinant protein of the present disclosure may be an expressed protein or an assembled protein.

As used herein, the term "antibody" or "immunoglobulin" is a tetraheteroglycan protein of about 150,000 Daltons having the same structural characteristics, consisting of two identical light chains (L) and two identical heavy chains (H). Each light chain is linked to the heavy chain by a covalent disulfide bond, and the number of disulfide bonds varies between heavy chains of different immunoglobulin isotypes. Each heavy chain and light chain also has regularly spaced intrachain disulfide bonds. Each heavy chain has a variable region (VH) at one end followed by a number of constant regions. Each light chain has a variable region (VL) at one end and a constant region at the other end; the constant region of the light chain is opposite the first constant region of the heavy chain, and the variable region of the light chain is opposite the variable region of the heavy chain. Particular amino acid residues form the interface between the variable regions of the light chain and heavy chain.

As used herein, the term "antigen-binding fragment" or "fab fragment" or "fab" consists of domains of the variable region of the light chain (VL), the constant region of the light chain (CL), the variable region of the heavy chain (VH), and the constant region 1 of the heavy chain (CH1), and may bind to the antigen. When referring to the variable region and constant region of the antigen-binding fragment being directly connected or connected via a linker (Linker), the constant region refers to the constant region of the light chain (CL) or the constant region 1 of the heavy chain (CH1).

As used herein, the term "first functional antigen" or "antigen of interest" refers to an antigen to which the first functional binding fragment binds.

As used herein, the term "second functional antigen" refers to a protein to which the second functional binding fragment binds.

As used herein, the term "Fc region" (fragment crystallizable, Fc) consists of the IgG constant regions CH2 and CH3 domains and the hinge region.

As used herein, the term "knobs-into-holes technology" or "knobs-into-holes" is using genetic engineering techniques to introduce different mutations in two CH3 domains of the heavy chain, thereby promoting the heterodimerization of the heavy chain. A knob is made on one heavy chain and a hole is made on the other heavy chain, and then the two preferentially couple together to form an asymmetric antibody (Ridgway JB, et al. "Knobs-into-holes" engineering of antibody CH3 domains for heavy chain heterodimerization. Protein Engineering, 1996, 9(7):617-621). As known to those skilled in the art, a plurality of knobs and/or holes can be made on one heavy chain, and correspondingly, a plurality of holes and/or knobs can also be made on the other heavy chain.

As used herein, the term "mutant" refers to a functional protein or functional fragment having an amino acid sequence different from that of the wild-type functional protein, for example, a new functional protein or functional fragment formed by insertions, deletions or substitutions of one or more amino acids on the functional protein or functional fragment. The mutant of the second functional binding fragment in the bispecific recombinant protein herein specifically refers to the mutant whose binding affinity to the corresponding second functional antigen is not higher than the binding affinity of the first functional binding fragment of the bispecific recombinant protein to the first functional antigen.

As used herein, the term "substitution" when applied to amino acid residues refers to the substitution of one or more amino acids, naturally occurring or introduced, with another in a peptide, polypeptide or protein to form a new peptide, polypeptide or protein. Substitutions in a polypeptide or protein may result in enhanced, decreased or unchanged function of the polypeptide or protein. Substitutions may also be "conservative substitutions," which, when directed to an amino acid sequence, refer to the substitution of one amino acid residue with another different amino acid residue in a side chain with similar physicochemical properties, or the substitution of those amino acids not critical to the activity of the polypeptide. For example, conservative substitutions may occur between the non-polar side chain amino acid residues (e.g., Met, Ala, Val, Leu and Ile, Pro, Phe, Trp), between the uncharged polar side chain residues (e.g., Cys, Ser, Thr, Asn, Gly and Gln), between the acidic side chain residues (e.g., Asp, Glu), between the basic side chain amino acids (e.g., His, Lys and Arg), between beta branched side chain amino acids (e.g., Thr, Val and Ile), between the sulfur-containing side chain amino acids (e.g., Cys and Met) or between the aromatic side chain residues (e.g., Trp, Tyr, His, and Phe). In some embodiments, the substitutions, deletions or additions may also be considered as "conservative substitutions". The number of amino acids inserted or deleted can range from about 1 to 5. Conservative substitutions generally do not cause significant changes in the conformational structure of the protein, and thus preserve the biological activity of the protein.

As used herein, the term "double-positive expressing cell" or "target cell" or "target cell of interest" refers to a cell that can interact with the first functional binding fragment and the second functional binding fragment simultaneously.

As used herein, the term "second functional antigen single-positive cell" or "non-target cell" or "non-target cell of interest" refers to a cell that does not interact with the first functional binding fragment, but only with the second functional binding fragment.

As used herein, the term "SIRPα" is Signal Regulatory Protein α, also known as CD172a. Signal Regulatory Protein (SIRP) is a transmembrane glycoprotein that includes three family members, SIRPα (CD172a), SIRPβ (CD172b), and SIRPγ (CD172g). These three members have similar extramembrane ends, but distinct intramembrane regions. The extramembrane end contains three immunoglobulin (Ig)-like regions, of which the first region belongs to the IgV region, and the second and third regions belong to the IgC region. The intramembrane region of SIRPα (CD172a) contains two inhibitory signal regions, which can transmit inhibitory signals and inhibit the corresponding functions of cells. The intramembrane regions of SIRPβ (CD172b) and SIRPγ (CD172g) are short and have no signal transduction regions, but SIRPβ (CD172b) can transmit activation signals through linker proteins (such as DAP12). SIRP proteins are mainly expressed in macrophages, dendritic cells (DC), and neuronal cells. Herein SIRPα specifically refers to human wild-type SIRPα and its CD47 non-high-affinity mutant.

As used herein, the terms "D1," "D2," and "D3" refer to the three extracellular Ig-like domains of SIRPα, which are successively D1 domain (Ig variable region-like domain, IgV region), D2 domain (Ig constant region-like domain, IgC region) and D3 domain (Ig constant region-like domain, IgC region) starting from the N-terminus of the protein, (Lee WY, et al. The Role of cis Dimerization of Signal Regulatory Proteina (SIRPα) in Binding to CD47. J Biol Chem, 2010, 285(49):37953-37963).

As used herein, the term "SIRPα-Fc fusion protein" refers to a fusion protein comprising a SIRPα extracellular truncated variant, a linker, and an Fc region. The linkers and/or Fc regions contained in the above sequences may be arbitrarily replaced according to methods well known to those skilled in the art or with commonly used linkers and/or Fc regions.

As used herein, the term "IFNα" or "IFN-α", i.e. "Interferon-α" or "Interferon α", comprises all natural or recombinant type α interferons, and is a member of type I interferons. It comprises 13 subtypes, such as IFN-α1a, IFN-α1b, IFN-α2a, IFN-α2b, IFN-α4a, IFN-4b, IFN-α5, IFN-α6, IFN-α7, IFN-α8, IFN-α10, IFN-α14, IFN-α16, IFN-α17, and IFN-α21. In the present disclosure, the term "IFNα" also comprises any substance having the biological activity of IFNα, for example, mutated or modified IFNα, such as PEG derivatives of IFNα (PEG-IFNα). In the present disclosure, the term "IFNα" is not limited to any particular acquisition source, and can be obtained from commercially available sources or produced by conventional techniques known to those skilled in the art. The methods of production include but not limited to biological source extraction methods and genetic engineering extraction methods, which are described in detail, for example, in Pestka S. Arch Biochem Biophys. 1983 Feb 15; 221(1): 1-37. In some embodiments, the IFNα is from a species selected from human, horse, cattle, mouse, pig, rabbit, cat, dog, rat, goat, sheep, and non-human primate. Particularly preferably, it is a human type α interferon.

As used herein, the term "IFNα 2b" or "IFN-α2b" or "IFNα2b" or "Interferon α2b" or "Interferon-α2b" is a subtype of IFN-α, all referring to Interferon-α2b.

As used herein, the term "IFN-γ," namely Interferon-γ, is a water-soluble dimeric cytokine and the only member of type II interferon. IFN-γ comprises a core and fragment sequences extended from the C-terminus domain consisting of six α-helices, and often forms a homodimer with two anti-parallel interlocking monomers to exert biological activity.

As used herein, the term "IFN-β" or "IFNβ," namely β-interferon (Interferon-β), belongs to human fibroblast interferons, and is also one of type I interferons, which is produced by various cells, such as fibroblasts, after induction by viruses, nucleic acids, etc. IFNβ binds to the same interferon receptors as IFNα, and has similar biological effects.

As used herein, the term "IFN-γ fusion protein" refers to a fusion protein comprising an IFN-γ truncated variant or an IFN-γ mutant, a linker and an Fc region. The linkers and/or Fc regions contained in the above sequences may be arbitrarily replaced according to methods well known to those skilled in the art or with commonly used linkers and/or Fc regions.

As used herein, the term "IL-10" or "IL10" refers to interleukin 10, and "IL-10M" or "IL10M" refers to an IL10 monomer mutant which can activate downstream signaling pathways without the need for homodimerization (Josephson, K. Design and analysis of an engineered human interleukin-10 monomer. [J]. Journal of Biological Chemistry, 2000, 275(18): 13552-7.).

As used herein, the term "IL-12" or "IL12" refers to interleukin 12, a heterodimeric molecule consisting of an α chain (p35 subunit, IL-12p35) and a β chain (p40 subunit, IL-12p40). The chains are covalently linked by disulfide bonds to form a biologically active 74 kDa heterodimer. As used herein, the term "IL12A" or "IL-12A" refers to the α chain of IL-12.

As used herein, the term "IL-15" or "IL15" refers to interleukin 15, a key cytokine in the activity of NK, NKT cells and CD8 memory T lymphocytes, which plays a role through the receptor consisting of three subunits called α, β, and γ, wherein the subunit α is unique to the IL15 receptor. As used herein, the term "IL-15RαSUSHI" or "IL15RαSUSHI" refers to the SUSHI domain on the α chain of the IL-15 receptor, which has its ordinary meaning in the art. The SUSHI domain affects IL15 to activate the downstream signaling pathways.

As used herein, the term "a complex formed by IL-15 and IL15RαSUSHI" comprises a fusion protein formed by IL15 and IL-15RαSUSHI, or a protein combination formed by combination of IL15 and IL-15RαSUSHI according to various physical or chemical means such as ionic bonds, covalent bonds, van der Waals force, etc.

As used herein, the term "linker sequence" or "Linker" refers to an amino acid sequence that links different functional binding fragments (e.g., a first functional binding fragment and a second functional binding fragment, a first functional binding fragment or a second functional binding fragment and an Fc region), or links different domains within the same functional binding fragment.

As used herein, the terms "Ofa", "Ofatumumab" and "Anti-CD20 (Ofatumumab)" are used interchangeably in the present disclosure to refer to the anti-CD20 antibody Ofatumumab.

As used herein, the terms "GC33" and "Codrituzumab" are used interchangeably in the present disclosure to refer to the anti-GPC3 antibody Codrituzumab.

As used herein, the terms "atezolizumab" and "Atezolizumab" are used interchangeably in the present disclosure to refer to the anti-PD-L1 antibody Atezolizumab.

As used herein, the term "host cell" generally comprises a single cell, cell line or cell culture that can be or has been the recipient of a subject plasmid or vector, which contains the polynucleotides in the present disclosure, or expresses the protein heterodimers of the present disclosure (e.g., heterodimeric proteins). Host cells can comprise a progeny of a single host cell. The progeny may not necessarily be identical (either morphologically or in genome total DNA complement) to the original parental cell due to natural, accidental or intentional mutation. Host cells can comprise cells transfected *in vitro* with the vectors in the present disclosure. Host cells can be bacterial cells (e.g., *E. coli*), yeast cells, or other eukaryotic cells, such as HEK293 cells, COS cells, Chinese Hamster Ovary (CHO) cells, HeLa cells, or myeloma cells. In some embodiments, host cells are mammalian cells. In some embodiments, the mammalian cells are CHO cells.

As used herein, the term "vector" generally refers to a nucleic acid molecule which is capable of self-replication in a suitable host and transfers an inserted nucleic acid molecule into host cells and/or between host cells. The term may comprise vectors primarily used for the insertion of DNA or RNA into cells, vectors primarily used for replication of DNA or RNA, and expression vectors used for transcription and/or translation of DNA or RNA. The term further comprises vectors that provide more than one of the above-mentioned functions. An "expression vector" is a polynucleotide that can be transcribed and translated into a polypeptide when introduced into a suitable host cell. An "expression system" generally means a suitable host cell containing an expression vector capable of producing the desired expression yield.

As used herein, the term "cell proliferation" or "proliferation" generally refers to the phenomenon in which the number of cells changes due to division. For example, cell proliferation can lead to an increase in the number of cells. The term further comprises cell growth by which cell morphology has been changed (e.g., increased in size), which is consistent with proliferative signals.

As used herein, the term "proliferation inhibition" or "inhibition of cell proliferation" generally refers to a reduction in the growth rate and/or proliferation rate of cancer cells. For example, this may comprise the death of cancer cells (e.g., by apoptosis). In some embodiments, the term may further refer to inhibiting the growth and/or proliferation of solid tumors and/or inducing size reduction or elimination of tumors.

As used herein, the terms "treatment", "therapy" and "medical treatment" are used interchangeably. The term "treatment" includes controlling the progression of a disease, disorder, or condition and associated symptoms, preferably reducing a disease, disorder, or condition, or alleviating the effects of one or more symptoms of a disease, disorder, or condition. The term includes cure of the disease or complete elimination of symptoms. The term includes remission of symptoms. The term also includes, but is not limited to, non-curative palliative treatment. The term "treatment" includes administering a therapeutically effective amount of a pharmaceutical composition comprising a recombinant protein or a fusion protein of the present disclosure to a subject, so as to prevent or delay, alleviate or relieve the progression of a disease, disorder, and condition, or the effects of one or more symptoms of a disease, disorder, and condition.

As used herein, the term "administration" refers to the delivery of a therapeutically effective amount of a pharmaceutical composition comprising a recombinant protein or a fusion protein of the present disclosure to a subject. The administration can be systemic or topical. The administration can be performed with an administration device, such as a syringe. Modes of administration include, but are not limited to, embedding, snorting, spraying, injecting, and the like. Routes of administration include inhalation, intranasal, oral, intravenous, subcutaneous or intramuscular administration, and the like.

Compared with the prior arts, the present disclosure has the following beneficial effects:

In the present disclosure, the C-terminus of the CL domain or the C-terminus of the CH1 domain in the antigen-binding fragment (fab segment) of the first functional binding fragment targeting the antigen of interest of the bispecific recombinant protein is directly connected to or connected via a linker to the second functional binding fragment targeting the immune checkpoint or the immune checkpoint ligand or the cytokine receptor, at the same time, the C-terminus of the second functional binding fragment is directly connected to or connected via a linker to the N-terminus of the Fc region, reducing the impurities, including the monomer or homodimer or homomultimer structure of the second functional binding fragment, that can target non-target cell of interest immune checkpoints, or immune checkpoint ligands, or cytokine receptors, generated during the preparation of the bispecific recombinant protein, unexpectedly reducing potential related safety risks caused by impurities of the product-related recombinant protein mixture (potentially risky impurities) in the fermentation product, and improving the convenience of recombinant protein preparation.

In the present disclosure, the C-terminus of the CL domain or the C-terminus of the CH1 domain in the antigen-binding fragment (fab segment) of the first functional binding fragment targeting the antigen of interest of the bispecific recombinant protein is directly connected to or connected via a linker to the second functional binding fragment targeting the immune checkpoint or the immune checkpoint ligand or the cytokine receptor, at the same time, the C-terminus of the second functional binding fragment is directly connected to or connected via a linker to the N-terminus of the Fc region, unexpectedly balancing and coordinating the safety and efficacy of the two targets, through mechanisms such as spatial conformation restriction, significantly reducing the binding of the bispecific recombinant protein to the immune checkpoints, or the immune checkpoint ligands, or the non-target cells of interest highly expressed with single-positive cytokine receptors or the cytokine receptors (the second functional antigen), improving the targeting of the bispecific recombinant protein, reducing the immune-related side effects such as potential cytokine storm caused by the targeting binding of non-target cells of interest with common symmetric or asymmetric bi-clonal antibody structures, and improving the safety of drugs. Unexpectedly, while maintaining the efficacy or superior efficacy of the first functional binding fragment of the bispecific recombinant protein in the target cells of interest (double-positive expressing cells), the bispecific recombinant protein, compared with the monomer/homodimer/homomultimer of the second functional binding fragment contained, significantly reduces the binding to the second functional antigen single-positive cells (non-target cells of interest) or even does not bind thereto, but at the same time, the interaction between the bispecific recombinant protein and the second functional antigen on the double-positive expressing cells (i.e. target cells of interest) is unexpectedly not weaker, or even significantly stronger than the monomer/homodimer/multimer of the second functional binding fragment, that is, the bispecific recombinant protein technology of the present disclosure can significantly reduce the related toxic side effects caused by the binding of the second functional binding fragment to the second functional antigen of the non-target cell of interest, and at the same time can strengthen the binding effect of the second functional binding fragment to the second functional antigen of the target cell of interest, and significantly improve the efficacy of the second functional binding fragments on target cells. For example, when a SIRPα extracellular truncated variant or a non-high-affinity mutant is selected as the second functional binding fragment, compared with SIRPα-Fc fusion protein (such as TTI-621), the bispecific recombinant protein of the present disclosure can significantly reduce its interaction with the CD47 single-positive non-target cells of interest such as red blood cells or platelets, but at the same time, the bispecific recombinant protein of the present disclosure can significantly improve the interaction with double-positive expressing cells (target cells of interest), significantly improving the killing effect of immune cells (such as macrophages) on target cells. And at the same time, the competitive binding ability of the bispecific recombinant protein of the present disclosure to the target cells of interest is significantly stronger than that of SIRPα-Fc fusion protein.

In addition, the bispecific recombinant protein of the present disclosure can also improve the freeze-thaw stability of some fusion proteins with immunoregulatory function. For example, when the second functional fragment is IFN-α 2b or its functional truncated variant or mutant, after repeated freezing and thawing for 5 times, the purity is above 95%, the appearance is clear, and the freeze-thaw stability is obviously better than that of IFN-α 2b monomer or PEGylated IFN-α 2b.

The bispecific recombinant protein of the present disclosure has strong extensibility in structure and simple design in screening. The first functional binding fragment can be selected from various antibody sequences, and the second functional binding fragment can be selected from cytokines, immune checkpoints or immune checkpoint ligand proteins and truncated variants thereof or mutants thereof, significantly reducing the time spent in conventional antibody drug screening, improving drug screening efficiency, and reducing screening costs. Optionally, the second functional binding fragment can be selected from cytokines and/or immune checkpoint binding proteins and/or immune checkpoint ligand binding proteins or truncated variants thereof; preferably, the second functional binding fragment can be selected from endogenous cytokines and/or immune checkpoint binding proteins and/or immune checkpoint ligand binding proteins or truncated variants thereof, so as to reduce the potential immunogenicity.

### BRIEF DESCRIPTION OF THE DRAWINGS

FIG. 1 shows a structural diagram of the bispecific recombinant protein of the present disclosure.
FIG. 2 shows SDS-PAGE electrophoresis diagrams of the bispecific recombinant protein in Embodiment 2 of the present disclosure after purification by protein A.
FIG. 3 shows non-reducing SDS-PAGE electrophoresis diagrams of the bispecific recombinant protein in Embodiment 2 of the present disclosure after affinity capture.
FIG. 4 shows a reducing SDS-PAGE electrophoresis diagrams of the bispecific recombinant protein in Embodiment 2 of the present disclosure after affinity capture.
FIG. 5 shows non-reducing SDS-PAGE electrophoresis diagrams of the bispecific recombinant protein in Embodiment 2 of the present disclosure after secondary purification.
FIG. 6 shows a binding curve of the bispecific recombinant protein of the present disclosure to CD20 single-positive cells (non-target cells of interest, CHO-K1-hCD20) determined by flow cytometry in Embodiment 3 of the present disclosure.
FIG. 7A shows a binding curve of the bispecific recombinant protein whose second functional antigen is CD47 of the present disclosure and the control sample to CD47 single-positive cells (non-target cells of interest, HEK293 cells) determined by flow cytometry in Embodiment 3 of the present disclosure.
FIG. 7B shows a binding curve of the bispecific recombinant protein whose second functional antigen is CD47 of the present disclosure and the control sample to CD47 single-positive cells (non-target cells of interest, CHO-K1 cells) determined by flow cytometry in Embodiment 3 of the present disclosure.
FIG. 8 shows a binding curve of the bispecific recombinant protein whose first functional antigen is CD20 and whose second functional antigen is CD47 of the present disclosure and the control sample to CD20/CD47 double-positive cells (target cells of interest, Raji cells) determined by flow cytometry in Embodiment 3 of the present disclosure.
FIG. 9A shows a competitive binding curve of the bispecific recombinant protein whose first functional antigen is CD20 and whose second functional antigen is CD47 of the present disclosure, the corresponding potentially risky impurity protein, and the control sample to CD20/CD47 double-positive cells (target cells of interest, Raji cells) determined by flow cytometry in Embodiment 3 of the present disclosure.
FIG. 9B shows a competitive binding curve of the bispecific recombinant protein whose first functional antigen is EpCAM and whose second functional antigen is CD47 of the present disclosure and the control sample to EpCAM/CD47 double-positive cells (target cells of interest, CAPAN-2 cells) determined by flow cytometry in Embodiment 3 of the present disclosure.
FIG. 9C shows a competitive binding curve of the bispecific recombinant protein whose first functional antigen is CD24 and whose second functional antigen is CD47 of the present disclosure and the control sample to CD24/CD47 double-positive cells (target cells of interest, MCF-7 cells) determined by flow cytometry in Embodiment 3 of the present disclosure.
FIG. 9D shows a competitive binding curve of the bispecific recombinant protein whose first functional antigen is CD38 and whose second functional antigen is CD47 of the present disclosure and the control sample to CD38/CD47 double-positive cells (target cells of interest, Raji cells) determined by flow cytometry in Embodiment 3 of the present disclosure.
FIG. 10 shows a binding activity curve of the bispecific recombinant protein whose first functional antigen is GPC3 and whose second functional antigen is IFN-α 2b in Embodiment 4 of the present disclosure and the control sample to target cells of interest, HepG2 hepatoma cells, determined by flow cytometry.
FIG. 11 shows a binding activity histogram of the bispecific recombinant protein whose first functional antigen is GPC3 and whose second functional antigen is IFN-α 2b in Embodiment 4 of the present disclosure and the control sample to target cells of interest HuH-7 determined by flow cytometry.
FIG. 12 shows an ADCC activity curve of the bispecific recombinant protein in Embodiment 5 of the present disclosure to target cells of interest HepG2 determined by LDH method.
FIG. 13 shows a proliferation inhibition activity curve of the bispecific recombinant protein with different linkers in Embodiment 6 of the present disclosure to target cells of interest HUH-7.
FIG. 14 shows a proliferation inhibition activity curve of the bispecific recombinant protein having the GPC3 antigen targeting function in Embodiment 6 of the present disclosure and the control sample to GPC3-positive target cells of interest HuH-7.
FIG. 15 shows a proliferation inhibition activity curve of the bispecific recombinant protein in Embodiment 7 of the present disclosure to PD-L1-positive target cells of interest MDA-MB-231.
FIG. 16 shows a proliferation inhibition activity curve of the bispecific recombinant protein in Embodiment 7 of the present disclosure to MDA-MB-231 blocked with anti-PD-L1 antibody.
FIG. 17A shows a proliferation inhibition activity curve of the bispecific recombinant protein in Embodiment 8 of the present disclosure to CD38-positive target cells of interest Daudi.
FIG. 17B shows a proliferation inhibition activity curve of the bispecific recombinant protein in Embodiment 8 of the present disclosure to CD38-negative non-target cells of interest SK-BR3.
FIG. 18 shows a proliferation inhibition activity curve of the bispecific recombinant protein containing different IFN-α 2b low-affinity mutants in Embodiment 9 of the present disclosure to GPC3-positive target cells of interest HuH-7.
FIG. 19 shows a proliferation inhibition activity curve of the bispecific recombinant protein containing different IFN-α 2b low-affinity mutants in Embodiment 9 of the present disclosure to GPC3-negative non-target cells of interest SW480.
FIG. 20 shows a proliferation inhibition activity curve of the bispecific recombinant protein containing different IFN-α 2b low-affinity mutants in Embodiment 9 of the present disclosure to GPC3-negative non-target cells of interest U266.
FIG. 21 shows a proliferation inhibition activity curve of the potentially risky impurities of the bispecific recombinant protein in Embodiment 10 of the present disclosure to GPC3-negative non-target cells of interest MDA-MB-231.
FIG. 22 shows a binding activity curve of the bispecific recombinant protein in Embodiment 11 of the present disclosure to TIGIT-positive target cells of interest H_IL12 Reporter 293 blocked with anti-TIGIT antibody.
FIG. 23 shows P-STAT3 activation level assay results of the bispecific recombinant protein in Embodiment 12 of the present disclosure to THP1 cells.
FIG. 24 shows proliferation activity assay results of the bispecific recombinant protein in Embodiment 13 of the present disclosure to PD-1-positive hPBMC stimulated with OKT3 for 48 hours.
FIG. 25 shows proliferation activity assay results of the bispecific recombinant protein in Embodiment 13 of the present disclosure to PD-1-negative non-target cells of interest M-07e.

### DETAILED DESCRIPTION OF THE PREFERRED EMBODIMENT

### Embodiment 1: Construction of expression vector

The bispecific recombinant protein was directly synthesized by GENEWIZ after codon optimization according to the protein sequence, and was inserted into the pCDNA3.1 plasmid, which was confirmed by sequencing. The above-mentioned different expression plasmids were mixed and paired to transfect expressing cells to obtain bispecific recombinant proteins or control samples (see Table 1). Subsequent experimental materials were extracted and obtained from expressing cells transfected with this series of plasmids.

**Table 1: Exemplary molecular structures of bispecific recombinant proteins and control samples**

| Bispecific recombinant protein | | | |
|---|---|---|---|
| Number of bispecific recombinant protein | First functional antigen×second functional binding fragment | Exemplary bi-clonal antibody molecular constitution | |
| | | chain A | chain B |
| LCB-001 | CD20×SIRPαD1 | Ofatumumab(H)-Fc1 | Ofatumumab(L)-SIRPaD 1 - Fc2 |
| LCB-002 | CD20×SIRPαD1 | Ofatumumab(H)-Fc1 | Ofatumumab(L)-(GGGGS)₃-SIRPaD 1-Fc2 |
| LCB-003 | CD20×SIRPαD1 | Ofatumumab(H)-(GGGS)₃-SIRPaD 1-Fc 1 | Ofatumumab(L)-Fc2 |
| LCB-004 | EGFR×SIRPαD1 | Panitumumab(H)-Fc1 | Panitumumab(L)-(GGGGS)₃-SIRPaD1-Fc2 |
| LCB-005 | EpCAM×SIRPα D1 | Edrecolomab (H)-Fc1 | Edrecolomab (L)-(GGGGS)₅-SIRPαD1-Fc2 |
| LCB-006 | CD24×SIRPaD 1 | SWA11(H)-Fc1 | SWA11(L)-(GGGGS)₂-SIRPaD 1-Fc2 |
| LCB-007 | AFP×IFNα 2b | Tacatuzumab(H)-Fc1 | Tacatuzumab(L)-(GGGGS)₃-IFNα2b-Fc2 |
| LCB-008 | AFP×IFNβ | Tacatuzumab(H)-Fc1 | Tacatuzumab(L)-(GGGGS)₃-IFNβ-Fc2 |
| LCB-009 | GPC3×IFN-α 2b | GC33(H)-Fc1 | GC33(L)-(GGGGS)₁-IFNα 2b-Fc2 |
| LCB-010 | GPC3×IFN-α 2b | GC33(H)-Fc1 | GC33(L)-(GGGGS)₂-IFNa 2b-Fc2 |
| LCB-011 | GPC3×IFN-α 2b | GC33(H)-Fc1 | GC33(L)-(GGGGS)₄-IFNα 2b-Fc2 |
| LCB-010-M1 | GPC3×IFN-α 2b | GC33(H)-Fc1 | GC33(L)-(GGGGS)₂-IFNa 2b(L26A)-Fc2 |
| LCB-010-M2 | GPC3×IFN-α 2b | GC33(H)-Fc1 | GC33(L)-(GGGGS)₂-IFNa 2b(L30A)-Fc2 |
| LCB-010-M3 | GPC3×IFN-α 2b | GC33(H)-Fc1 | GC33(L)-(GGGGS)₂-IFNa 2b(A145G)-Fc2 |
| LCB-010-M4 | GPC3×IFN-α 2b | GC33(H)-Fc1 | GC33(L)-(GGGGS)₂-IFNa 2b(R149A)-Fc2 |
| LCB-010-M5 | GPC3×IFN-α 2b | GC33(H)-Fc1 | GC33(L)-(GGGGS)₂-IFNa 2b(S152A)-Fc2 |
| LCB-011-M3 | GPC3×IFN-α 2b | GC33(H)-Fc1 | GC33(L)-(GGGGS)₄- IFNα 2b(A145G)-Fc2 |
| LCB-011-M4 | GPC3×IFN-α 2b | GC33(H)-Fc1 | GC33(L)-(GGGGS)₄-IFNα 2b(R149A)-Fc2 |
| LCB-012 | RSV×IFN-α2b | Palivizumab(H)- Fc1 | Palivizumab (L)-(GGGGS)₂-IFNα2b -Fc2 |
| LCB-013 | PD-L1×IFN-α2b | Atezolizumab (H)-Fc1 | Atezolizumab (L)-(GGGGS)₁-IFNα2b-Fc2 |
| LCB-014 | PD-L1×IFN-α2b | Atezolizumab (H)-Fc1 | Atezolizumab (L)-(GGGGS)₂-IFNα 2b-Fc2 |
| LCB-015 | PD-L1×IFN-α2b | Atezolizumab (H)-Fc1 | Atezolizumab (L)-(GGGGS)₃-IFNα 2b-Fc2 |
| LCB-016 | CD38×IFN-α 2b | Mezagitamab(L)-Fc1 | Mezagitamab (H)-(GGGGS)₅-IFNα2b-Fc2 |
| LCB-017 | CD38×SIRPaD 1 | Felzartamab(H)-Fc2 | Felzartamab(L)-(GGGGS)₅-SIRPaD 1-Fc 1 |
| LCB-018 | TIGIT×IL-12 | Tiragohimab(H)-Fc1 | Tiragolumab (L)-(GGGGS)₄-IL12A-Fc2 |
| LCB-019 | CD80×IL-10 | Galiximab(H)-Fc1 | Galiximab(L)- (GGGGS)₅-IL10M-Fc2 |
| LCB-020 | PD-1×IL-15-IL15RaSUSHI | Nivolumab(H)-Fc1 | Nivolumab(L)-(GGGGS)₅-IL15-(GGGGS)₅-IL15RaSUSHI-Fc2 |
| LCB-021 | PD-1×IL-15-IL15RaSUSHI | Nivolumab(H)-Fc1 | Nivolumab(L)-(GGGGS)₅-IL15RαSUSHI-(GGGGS)₅-IL15-Fc2 |
| LCB-022 | RSV×IL-10 | Palivizumab(H)- Fc1 | Palivizumab (L)-(GGGGS)₅-IL10M-Fc2 |
| LCB-023 | RSV×IL-15-IL15RaSUSHI | Palivizumab(H)- Fc1 | Palivizumab (L)-(GGGGS)₅-IL15-(GGGGS)₅-IL15RaSUSHI-Fc2 |
| LCB-024 | RSV×IL-15-IL15RaSUSHI | Palivizumab(H)- Fc1 | Palivizumab (L)-(GGGGS)₅-IL15RαSUSHI-(GGGGS)₅-IL15-Fc2 |

| Control sample | | | |
|---|---|---|---|
| Name of control sample | Antigen | Molecular constitution | |
| Ofatumumab | CD20 | Anti-CD20 monoclonal antibody Ofatumumab | |
| Rituximab | CD20 | Anti-CD20 monoclonal antibody Rituximab | |
| Magrolimab | CD47 | Anti-CD47 monoclonal antibody Magrolimab | |
| Ofa-Fc1-D1-Fc2 | CD20×CD47 | Ofatumumab-Fc1, D1-Fc2 (see CN108864290A SEQ ID NO: 16 (Ofa-Fc1 heavy chain)+SEQ ID NO: 17 (Ofa-Fc1 light chain)+SEQ ID NO: 26 (D1-Fc2)) | |
| TTI-621 | CD47 | SIRPαD1-Fc homodimer (see CN105073780B SEQ ID NO: 25) | |
| SIRPα-D1m-Fc | CD47 | SIRPαD1m-Fc homodimer (D1m sequence, see CN108864290A SEQ ID NO: 33) | |
| LCB-001-R | CD47 | Ofatumumab(L)-SIRPαD1-Fc2 homodimer | |
| LCB-002-R | CD47 | Ofatumumab(L)-(GGGGS)₃-SIRPαD1-Fc2 homodimer | |
| Codrituzumab | GPC3 | Anti-GPC3 monoclonal antibody codrituzumab | |
| Palivizumab | Respiratory syncytial virus (RSV) | Anti-RSV monoclonal antibody Palivizumab | |
| Atezolizumab | PD-L1 | Anti-PD-L1 monoclonal antibody Atezolizumab | |
| Felzartamab | CD38 | Anti-CD38 monoclonal antibody Felzartamab | |
| Mezagitamab | CD38 | Anti-CD38 monoclonal antibody Mezagitamab | |
| Tiragolumab | TIGIT | Anti-TIGIT monoclonal antibody Tiragolumab | |
| Galiximab | CD80 | Anti-CD80 monoclonal antibody Galiximab | |
| Nivolumab | PD-1 | Anti-PD-1 monoclonal antibody Nivolumab | |
| IFN-α 2b-Fc | None | Recombinant expression of IFN-α 2b and human IgG1 Fc fusion protein | |
| IFN-α 2b | None | Recombinant expression of IFN-α 2b protein | |
| IL10M-Fc | None | Recombinant expression of human IL10 monomer mutant and human IgG1 Fc fusion protein | |
| IL 15-IL15RaSUSHI-Fc | None | Recombinant expression of human IL15, IL15RaSUSHI and human IgG1 Fc fusion protein | |
| Human IgG1 homotype control (Isotype) | Hen egg lysozyme (HEL) | Anti-HEL monoclonal antibody as homotype control | |

In the Table 1 above, the first functional binding fragment of the bispecific recombinant protein is characterized by the antigen of interest it targets, namely the first functional antigen; (H) refers to the domain consisting of heavy chains VH and CH1, (L) refers to the domain consisting of light chains VL and CL; D1 represents the extracellular D1 domain of wild-type human SIRPα and its mutants; Fc represents the wild-type Fc region, Fc1 represents the Fc region with hole or holes mutation, and Fc2 represents the Fc region with knob or knobs mutation. The corresponding sequence numbers of the sequence names are shown in Table 2. Herein, the sequence of the signal peptide is shown in SEQ ID NO: 49. The amino acid sequence of Codrituzumab is referenced from the patent US7919086, the amino acid sequence of the heavy chain is shown in SEQ ID NO: 19, and the amino acid sequence of the light chain is shown in SEQ ID NO: 20. The amino acid sequence of Atezolizumab is referenced from the patent US20100203056, the amino acid sequence of the heavy chain is shown in SEQ ID NO: 21, and the amino acid sequence of the light chain is shown in SEQ ID NO: 22. The amino acid sequence of the heavy chain of Tiragolumab is shown in SEQ ID NO: 61, and the amino acid sequence of the light chain is shown in SEQ ID NO: 62. The amino acid sequences of heavy chain variable region and light chain variable region of Palivizumab are referenced from the patent WO1994017105. Human IgG1 isotype control (B 117901) was purchased from Biointron. Recombinantly expressed IFN-α 2b protein (Z03003) was purchased from Nanjing GenScript Biotechnology Co., Ltd. IL10 in IL10M-Fc was in the form of a monomer mutant, and the corresponding sequence is referred from the following reference (Josephson, K. Design and analysis of an engineered human interleukin-10 monomer. [J]. Journal of Biological Chemistry, 2000, 275 (18): 13552-7.). IL15-IL15RaSUSHI-Fc (C15Y) was purchased from Novoprotein.

**Table 2: Sequence name and corresponding sequence number**

| Sequence name | Sequence number |
|---|---|
| Ofatumumab(H)-Fc1 | SEQ ID NO: 1 |
| Ofatumumab(L)-SIRPαD1-Fc2 | SEQ ID NO: 2 |
| Ofatumumab(L)-(GGGGS)₃-SIRPαD1-Fc2 | SEQ ID NO: 3 |
| GC33(H)-Fc1 | SEQ ID NO: 4 |
| GC33(L)-(GGGGS)₁-IFNα2b-Fc2 | SEQ ID NO: 5 |
| GC33(L)-(GGGGS)₂-IFNa 2b-Fc2 | SEQ ID NO: 6 |
| GC33(L)-(GGGGS)₄-IFNα 2b-Fc2 | SEQ ID NO: 7 |
| GC33(L)-(GGGGS)₂-IFNa 2b(L26A)-Fc2 | SEQ ID NO: 8 |
| GC33(L)-(GGGGS)₂-IFNα 2b(L30A)-Fc2 | SEQ ID NO: 9 |
| GC33(L)-(GGGGS)₂-IFNa 2b(A145G)-Fc2 | SEQ ID NO: 10 |
| GC33(L)-(GGGGS)₂-IFNα 2b(R149A)-Fc2 | SEQ ID NO: 11 |
| GC33(L)-(GGGGS)₂-IFNa 2b(S152A)-Fc2 | SEQ ID NO: 12 |
| GC33(L)-(GGGGS)₄-IFNα 2b(A145G)-Fc2 | SEQ ID NO: 13 |
| GC33(L)-(GGGGS)₄-IFNα 2b(R149A)-Fc2 | SEQ ID NO: 14 |
| Palivizumab(H)- Fc1 | SEQ ID NO: 15 |
| Palivizumab (L)-(GGGGS)₂-IFNα 2b-Fc2 | SEQ ID NO: 16 |
| IFN-α 2b-Fc1 | SEQ ID NO: 17 |
| IFN-α 2b | SEQ ID NO: 18 |
| GC33(H)-Fc | SEQ ID NO: 19 |
| GC33(L) | SEQ ID NO: 20 |
| Atezolizumab(H)-Fc | SEQ ID NO: 21 |
| Atezolizumab(L) | SEQ ID NO: 22 |
| Atezolizumab (H)-Fc1 | SEQ ID NO: 23 |
| Atezolizumab (L)-(GGGGS)₁-IFNα 2b-Fc2 | SEQ ID NO: 24 |
| Atezolizumab (L)-(GGGGS)₂-IFNα 2b-Fc2 | SEQ ID NO: 25 |
| Atezolizumab (L)-(GGGGS)₃-IFNα 2b-Fc2 | SEQ ID NO: 26 |
| Edrecolomab (H)-Fc1 | SEQ ID NO: 27 |
| Edrecolomab (L)-(GGGGS)₅- SIRPαD1-Fc2 | SEQ ID NO: 28 |
| SWA11(H)-Fc1 | SEQ ID NO: 29 |
| SWA11(L)-(GGGGS)₂- SIRPαD1-Fc2 | SEQ ID NO: 30 |
| Mezagitamab(L)-Fc 1 | SEQ ID NO: 31 |
| Mezagitamab (H)-(GGGGS)₅-IFNα2b-Fc2 | SEQ ID NO: 32 |
| Felzartamab(H)-Fc2 | SEQ ID NO: 33 |
| Felzartamab(L)-(GGGGS)₅- SIRPαD1-Fc1 | SEQ ID NO: 34 |
| Tiragolumab(H)-Fc 1 | SEQ ID NO: 35 |
| Tiragolumab (L)-(GGGGS)₄-IL12A-Fc2 | SEQ ID NO: 36 |
| Galiximab(H)-Fc1 | SEQ ID NO: 37 |
| Galiximab(L)- (GGGGS)₅-IL10M-Fc2 | SEQ ID NO: 38 |
| Nivolumab(H)-Fc1 | SEQ ID NO: 39 |
| Nivolumab(L)-(GGGGS)₅-IL15-(GGGGS)₅- IL15RαSUSHI-Fc2 | SEQ ID NO: 40 |
| Nivolumab(L)-(GGGGS)₅-IL15RαSUSHI-(GGGGS)₅-IL15-Fc2 | SEQ ID NO: 41 |
| Palivizumab (L)- (GGGGS)s-IL10M-Fc2 | SEQ ID NO: 42 |
| Palivizumab(L)-(GGGGS)₅-IL15-(GGGGS)₅-IL15RαSUSHI-Fc2 | SEQ ID NO: 43 |
| Palivizumab (L)-(GGGGS)₅-IL15RαSUSHI-(GGGGS)₅-IL15-Fc2 | SEQ ID NO: 44 |
| Felzartamab(H)-Fc | SEQ ID NO: 45 |
| Felzartamab(L) | SEQ ID NO: 46 |
| IL10M-Fc | SEQ ID NO: 47 |
| IL 12B | SEQ ID NO: 48 |
| Signal peptide | SEQ ID NO: 49 |
| SIRPαD1 | SEQ ID NO: 50 |
| IL-12A | SEQ ID NO: 51 |
| IL-10M | SEQ ID NO: 52 |
| IL-15 | SEQ ID NO: 53 |
| IL-15RαSUSHI | SEQ ID NO: 54 |
| IFN β | SEQ ID NO: 55 |
| Panitumumab (H)-Fc1 | SEQ ID NO: 56 |
| Panitumumab(L)-(GGGGS)₃-SIRPαD1-Fc2 | SEQ ID NO: 57 |
| Tacatuzumab(H)-Fc 1 | SEQ ID NO: 58 |
| Tacatuzumab(L)-(GGGGS)₃-IFNα2b-Fc2 | SEQ ID NO: 59 |
| Tacatuzumab(L)-(GGGGS)₃-IFNβ-Fc2 | SEQ ID NO: 60 |
| Ofatumumab(H)-(GGGS)₃-SIRPαD1-Fc1 | SEQ ID NO: 61 |
| Ofatumumab(L)-Fc2 | SEQ ID NO: 62 |
| Tiragolumab(H)-Fc | SEQ ID NO: 63 |
| Tiragolumab (L) | SEQ ID NO: 64 |

In Table 1, human IFN-α 2b (Genebank: AAP20099.1) was used as an example to illustrate the design of IFNα as a second functional binding fragment of the bispecific recombinant protein. There are 15 subtypes in the IFNα family. Different subtypes have similar structures, high sequence homology (80-99%), and bind to the same IFN receptors. All subtypes have the functions of antivirus, proliferation inhibition, antitumor and immunoregulation. Regarding the technical effects that can be achieved by IFN-α 2b, the other subtypes of IFN-α can achieve the identical technical effects (British Journal of Pharmacology (2013) 168 1048-1058), which will not be repeated here in the present disclosure. IFN-β and IFN-α 2b have the same interferon receptor and are both type I interferons and have similar biological effects, therefore they can also achieve the identical technical effects.

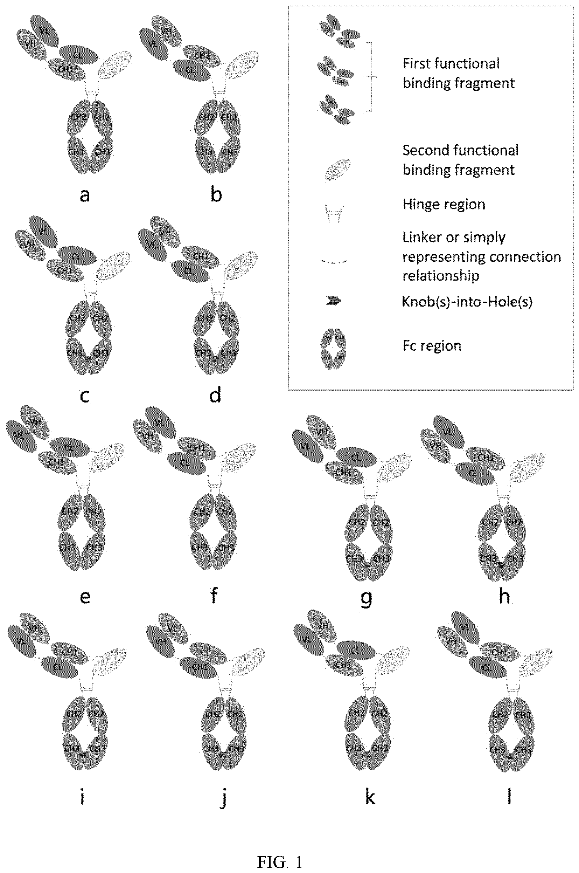

The structure of the bispecific recombinant protein of the present disclosure is shown in FIG. 1, the first functional binding fragment targeting the antigen of interest of the bispecific recombinant protein comprises an antigen-binding fragment, wherein the C-terminus of the CL domain and the C-terminus of the CH1 domain in the antigen-binding fragment is directly connected to or is connected via a linker to the second functional binding fragment targeting the immune checkpoint or the immune checkpoint ligand or the cytokine receptor. The variable region (V region) and the constant region (C region) in the antigen-binding fragment of the first functional binding fragment are directly connected or are connected via a linker; or the antigen-binding fragment is directly connected to or is connected via a linker to the Fc region; or both above-mentioned methods are used simultaneously to connect. The light chain VL or heavy chain VH of the first functional binding fragment is bound to the second functional binding fragment via knobs-into-holes. The light chain VL or heavy chain VH of the first functional binding fragment or the second functional binding fragment is directly connected to or is connected via a linker to the Fc region. For example, in FIG. 1, a and e represent that the C-terminus of the CL domain of the antigen-binding fragment of the first functional binding fragment is directly connected to or is connected via a linker to the second functional binding fragment; b and f represent that the C-terminus of the CH1 domain of the antigen-binding fragment of the first functional binding fragment is directly connected to or is connected via a linker to the second functional binding fragment; c, g, j, and k represent that the C-terminus of the CL domain of the antigen-binding fragment of the first functional binding fragment is directly connected to or is connected via a linker to the second functional binding fragment, and the Fc region is bound via knobs-into-holes; d, h, i, and 1 represent that the C-terminus of the CH1 domain of the antigen-binding fragment of the first functional binding fragment is directly connected to or is connected via a linker to the second functional binding fragment, and the Fc region is bound via knobs-into-holes.

### Embodiment 2: Preparation of expression plasmid, cell transfection, and expression and purification of target protein

### 1. Preparation of expression plasmid

A bacterial glycerol stock containing the expression plasmid (1 mL of the *E. coli* bacterial solution containing the expression plasmid was added with 0.5 mL of 60% sterile glycerol solution and mixed thoroughly) was inoculated into a liquid LB medium at a ratio of 1:1000. The bacteria were collected by centrifugation at 37°C, 220 rpm after culturing in a shaker for 16 hours. An endotoxin-free plasmid maxiprep kit (DP117, purchased from Tiangen Biotech (Beijing) Co., Ltd.) was used to extract the expression plasmid according to the standard procedure provided by the kit instruction.

### 2. Cell transfection and protein expression

The following method takes LCB-001 as an example, and is suitable for the bispecific recombinant protein whose second functional antigen is CD47.

After the obtained expression plasmid was filtered with a 0.22 µm filter membrane, 3 mg of the plasmid (wherein the ratio of expression plasmids of the chain A and chain B of the bispecific recombinant protein was 1:1 (molar ratio)) was added to 50 mL of Opti MEM I Reduced Serum Medium (GIBCO) and mixed thoroughly. 6 mg of transfection reagent polyetherimide (PEI, purchased from Polysciences, dissolved in sterile ultrapure water at a concentration of 1 mg/mL) was pipetted into 50 mL of Opti MEM I Reduced Serum Medium and mixed thoroughly. The obtained PEI solution was added to the Opti MEM I Reduced Serum Medium solution containing the plasmid and mixed thoroughly. After standing at room temperature for 15 minutes, the mixture of plasmid and PEI was slowly and evenly added to a suspension of host cell CHO-S (Thermo Fisher) with a volume of 1 L and a cell density of 3 ×10⁶ cells/mL, and cultured in a 37°C, 5% CO₂ incubator. After 4 hours, a feed medium (the formulation of the feed medium was 80 g of CD Efficient FeedC AGT (Gibco) and 75 g of 5×00483 (Kerry) dissolved in 1L of water) with a volume equivalent to 7% of the initial volume was added therein. The culture temperature was reduced to 33°C and the cells were harvested after 6 days of culture. The cell suspension was centrifuged at 10°C, 10,000 g for 30 minutes, and the supernatant obtained by centrifugation, i.e., the cell culture harvest solution, was used for purification of the target protein.

The following method takes LCB-009 as an example, and is suitable for the bispecific recombinant protein whose second functional antigen is other than CD47.

After the obtained expression plasmid was filtered with a 0.22 µm filter membrane, 50 µg of the plasmid (wherein the mass ratio of expression plasmids of the chain A and chain B was 2:1 or 3:1) was added to 2 mL of OptiPRO SFM Medium (GIBCO) and mixed thoroughly. 160 µL of transfection reagent ExpiFectamine CHO Reagent was pipetted into 2 mL of OptiPRO SFM Medium and mixed thoroughly. The obtained mixed solution of transfection reagent was added to the mixed solution containing plasmid and mixed thoroughly. The mixture of plasmid and transfection reagent was slowly and evenly added to a suspension of host cell ExpiCHO-S (Thermo Fisher) with a volume of 50 mL and a cell density of 6×10⁶ viable cells/mL, and cultured in a 37°C, 8% CO₂ incubator. On day 1 (after 18-22 hours), 300 µL of ExpiCHO Enhancer and 8 mL of ExpiCHO Feed were added, and the culture temperature was reduced to 32°C; on day 5, a second feed was performed, supplemented with 8 mL of ExpiCHO Feed, and the cells were harvested after 12 days. The cell suspension was centrifuged at 8000 rpm for 15 minutes, and the supernatant obtained by centrifugation, i.e., the cell culture harvest solution, was used for purification of the target protein.

### 3. Protein purification

### 1) Sample capture (Protein A affinity capture)

The following method takes LCB-001 as an example, and is suitable for all bispecific recombinant proteins of the present disclosure.

The above-mentioned cell culture harvest solution of LCB-001 was centrifuged at 10,000 rpm for 30 minutes to remove the cells and fragments thereof, then loaded onto a Protein A affinity column (GE Healthcare), and eluted to harvest the target protein. The purity of the protein was detected by SDS-PAGE.

The purification method of Protein A is a conventional protein purification method well known to those skilled in the art, the detailed method can be found in the GE Healthcare Protein A product instructions and GE Antibody Purification Manual.

### 2) Sample secondary purification

The following method takes LCB-009 as an example to illustrate the secondary purification steps of the bispecific recombinant protein, and is suitable for the bispecific recombinant protein containing many more aggregations after Protein A affinity capture.

SULFATE 650F packing (TOSOH) was used to remove the aggregations and other impurities in the sample of the expression supernatant of bispecific recombinant protein LCB-009. The experimental procedures are as follows:
a) equilibration: an equilibration solution (50mM NaAC-HAC, pH5.5) was used to equilibrate the column until the UV detection line is stable;
b) loading: the sample was loaded by the sample pump, the retention time was 5 min, and the loading capacity was ≤50mg/mL;
c) re-equilibration: the equilibration solution (50mM NaAC-HAC, pH5.5) was used to wash the chromatography column for 5 column volumes;
d) elution: an eluent (50 mM NaAC-HAC, 250 Mm, pH 5.5) was used to elute the target protein, and SDS-PAGE was used to detect the purity of the protein.

The purification steps of the control sample in Table 1 were obtained with reference to the operation steps of 1) Sample capture (Protein A affinity capture).

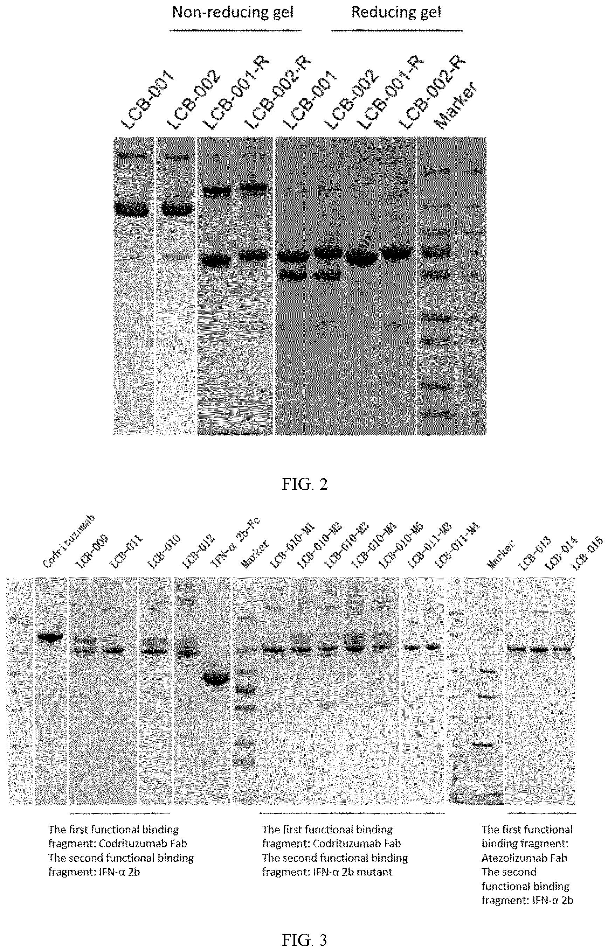

The SDS-PAGE protein electrophoresis assay results of the purified bispecific recombinant protein, the control sample and the potentially risky impurities are shown in FIG. 2-FIG. 5.

The theoretical molecular weights of the four proteins, LCB-001, LCB-002, LCB-001-R and LCB-002-R, are 111kD, 114kD, 123kD and 129kD, respectively. As shown in FIG. 2, the target proteins in each lane are normally expressed, but different degrees of left arm dimer, right arm dimer (LCB- 001-R, LCB-002-R) and/or multimer are observed in LCB-001 and LCB-002 (lane 1 and lane 2 in FIG. 2).

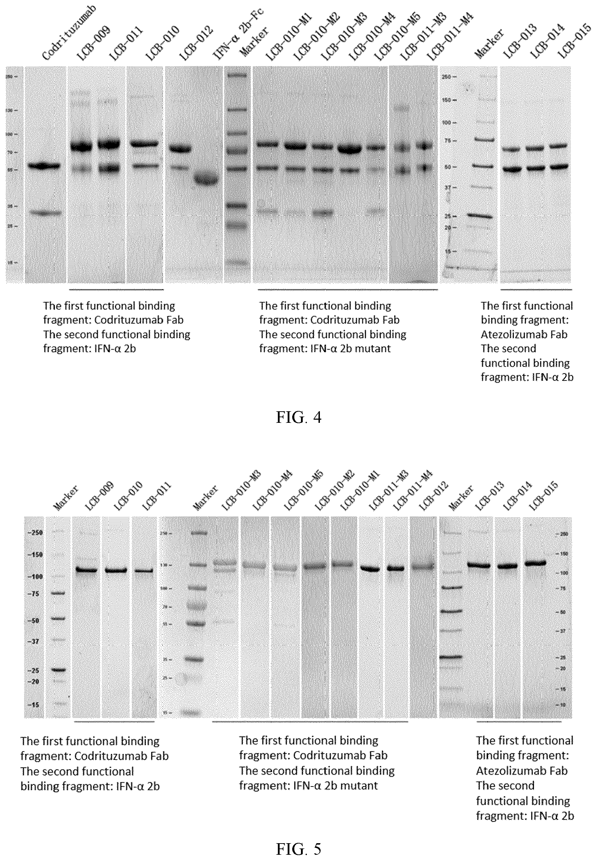

The theoretical molecular weights of LCB-009, LCB-010, LCB-011, LCB-012, LCB-013, LCB-014, LCB-015, LCB-010-M1, LCB-010-M2, LCB-010-M3, LCB-010-M4, LCB-010-M5, LCB-011-M3, and LCB-011-M4 are all about 120kD. The theoretical molecular weight of the control antibody Codrituzumab shown in Table 1 is 150kD, the molecular weight of IFNα 2b-Fc is 88kD, and the molecular weight of IFNα 2b monomer is 19.2kD. The non-reducing SDS-PAGE protein electrophoresis assay results of the affinity captured (i.e., purified by Protein A) samples are shown in FIG. 3, and the reducing SDS-PAGE protein electrophoresis assay results are shown in FIG. 4. The electrophoresis results show that the antibody expressed by this structure contains many more aggregations. The non-reducing SDS-PAGE protein electrophoresis assay results after secondary purification (i.e., purified by SULFATE 650F) are shown in FIG. 5. It can be seen that most of the aggregations may be removed by cation exchange chromatography packing.

After affinity capture, secondary purification of the other bispecific recombinant proteins shown in Table 1, the reducing and non-reducing SDS-PAGE protein electrophoresis assay results of the samples are consistent with the theoretical molecular weights, which will not be repeated herein.

### Embodiment 3: Assays of affinity and competitive binding activity to the target of the bispecific recombinant protein whose second functional antigen is CD47

### 1. Assay method for affinity of the target CD47 and/or CD20

### Determination of affinity of the bispecific recombinant protein to the target CD20 by flow cytometry:

The binding affinity of the bispecific recombinant protein to the target CD20 was determined by flow cytometry. The following method takes LCB-001 or LCB-002 as an example, and is suitable for the assay of the recombinant protein whose first functional antigen is CD20.

CHO-K1-hCD20 cells (hCD20-overexpressed Chinese hamster ovary epithelial cells) were cultured, well-grown cells were collected and counted, centrifuged and resuspended in PBS+2% FBS (purchased from Gibco) to a concentration of 3×10⁶ cells/mL. 100 µL of the cell suspension was aliquoted to each well of a 96-well U-shaped plate (Art. No. 3799, Corning), and allowed to stand for at least 15 minutes; the plate was centrifuged and the supernatant was aspirated, then 8 dilution gradients of LCB-001, LCB-002, positive control Ofatumumab, positive control Rituximab, or negative control IgG (Isotype) (3-fold serial dilutions starting from 100 nM with 8 concentrations in total) were added respectively, and the 96-well plate was incubated in a refrigerator at 4°C for 1 hour; after rinsing with PBS+2% FBS, goat anti-human IgG Fc-FITC (F9512-2ML, Sigma) was added and the plate was incubated at 4°C for 1 hour; after rinsing and resuspension with PBS+2% FBS, the fluorescence value was determined by flow cytometry (BD).

Since CHO-K1 cells do not express CD47 antigen, CHO-K1-hCD20 cells can be used to evaluate the binding affinity of the recombinant proteins LCB-001 and LCB-002, positive control samples Ofatumumab and Rituximab, and negative control IgG to CD20 at the cellular level.

The results show that, except that the negative control IgG cannot bind to CHO-K1, the recombinant proteins LCB-001 and LCB-002, and the positive control samples Ofatumumab and Rituximab can all bind to CHO-K1 cells; the binding affinity of LCB-001 and LCB-002 to CD20 is similar to that of the anti-CD20 antibody Ofatumumab or Rituximab.

The above experimental data prove that the recombinant protein of the present disclosure can specifically target the CD20 antigen of tumor cells at the cellular level, and the binding affinity to CD20 is not lower than the binding affinity of the monoclonal antibody with the same target to CD20; the recombinant protein of the present disclosure can target the target cells of interest with high affinity.

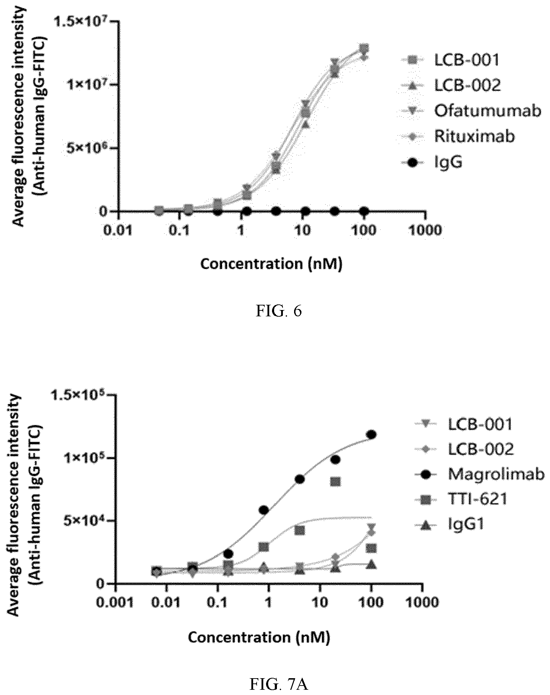

For example, as shown in FIG. 6, the recombinant proteins LCB-001 and LCB-002, the positive control samples Ofatumumab and Rituximab can all bind to CHO-K1-hCD20 cells. Specifically, the binding affinity of LCB-001 and LCB-002 to CD20 is similar to that of the anti-CD20 antibody Ofatumumab or Rituximab.

### Determination of affinity of the bispecific recombinant protein to the target CD47 by flow cytometry:

The binding affinity of the bispecific recombinant protein to the target CD47 was determined by flow cytometry. The following method takes LCB-001, LCB-002, or LCB-017 as an example, and is suitable for the assay of the recombinant protein whose second functional antigen is CD47.

HEK293 cells (human embryonic kidney cells 293) (CD20-/CD47+, non-target cells of interest) were cultured, well-grown cells were collected and counted, centrifuged and resuspended in PBS+2% FBS (Gibco) to a concentration of 3×10⁶ cells/mL. 100 µL of the cell suspension was aliquoted to each well of a 96-well U-shaped plate (Art. No. 3799, Corning), and allowed to stand for at least 15 minutes; the plate was centrifuged and the supernatant was aspirated, then 7 dilution gradients of LCB-001, LCB-002, anti-CD47 antibody Magrolimab, anti-CD47 fusion protein TTI-621 or IgG1 (Isotype) (4-fold serial dilutions starting from 100 nM with 7 concentrations in total) were added respectively, and the 96-well plate was incubated in a refrigerator at 4°C for 1 hour; after rinsing with PBS+2% FBS, goat anti-human IgG Fc-FITC (F9512-2ML, Sigma) was added and the plate was incubated at 4°C for 1 hour; after rinsing and resuspension with PBS+2% FBS, the fluorescence value was determined by flow cytometry (BD).

Since HEK293 cells do not express CD20 antigen, HEK293 cells can be used to evaluate the binding affinity of the bispecific recombinant proteins LCB-001 and LCB-002, positive control anti-CD47 antibody Magrolimab, anti-CD47 fusion protein TTI-621, and negative control IgG to CD47 at the cellular level.

The results show that, except that the negative control IgG cannot bind to HEK293, the anti-CD47 antibody Magrolimab, anti-CD47 fusion protein TTI-621 (SIRPα D1-Fc fusion protein), recombinant proteins LCB-001 and LCB-002 can all bind to HEK293 cells; the binding of the recombinant proteins LCB-001 and LCB-002 to HEK293 is significantly weaker than that of the anti-CD47 antibody Magrolimab, and is also non-obviously weaker than that of the anti-CD47 fusion protein TTI-621.

For example, as shown in FIG. 7A, the anti-CD47 antibody Magrolimab, the anti-CD47 fusion protein TTI-621, the recombinant proteins LCB-001 and LCB-002 were all able to bind to HEK293 cells. Specifically, the affinity of the recombinant proteins LCB-001 and LCB-002 was highly significantly weaker than that of the anti-CD47 antibody Magrolimab, and significantly weaker than that of the anti-CD47 fusion protein TTI-621 (SIRPα D1-Fc fusion protein).

After CD47-transfected CHO-K1 cells (Chinese hamster ovary cells) (CD38-/CD47+, non-target cells of interest) were rinsed and digested, well-grown cells were collected and counted, centrifuged and resuspended in DPBS + 2% FBS (Gibco) to a concentration of 3×10⁶ cells/mL. 100 µL of the cell suspension was aliquoted to each well of a 96-well U-shaped plate (Art. No. 3799, Corning) and allowed to stand for at least 15 minutes; the plate was centrifuged and the supernatant was aspirated, then 7 dilution gradients of LCB-017, Felzartamab, TTI-621, or SIRPα-D1m-Fc (3-fold serial dilutions starting from 200 nM with 11 concentrations in total), and the 96-well plate was incubated in a refrigerator at 4°C for 1 hour; after rinsing with DPBS+2% FBS, goat anti-human IgG Fc-FITC (F9512-2ML, Sigma) was added and the plate was incubated at 4°C for 1 hour; after rinsing and resuspension with DPBS+2% FBS, the fluorescence value was determined by flow cytometry (BD).

Since CD47-transfected CHO-K1 cells do not express CD38 antigen, the CHO-K1 cells can be used to evaluate the binding affinity of the bispecific recombinant protein LCB-017, the negative control anti-CD38 antibody Felzartamab, and the positive control anti-CD47 fusion protein TTI-621 and its high-affinity SIRPα D1m-Fc to CD47 at the cellular level.

The results show that, except that the negative control anti-CD38 antibody Felzartamab cannot bind to CHO-K1, the anti-CD47 fusion protein TTI-621 (SIRPα D1-Fc fusion protein), high-affinity SIRPα D1m-Fc fusion protein and recombinant protein LCB-017 can all bind to HEK293 cells; the binding of the recombinant protein LCB-017 to non-target cell of interest CHO-K1 is significantly weaker than that of the anti-CD47 fusion protein TTI-621.

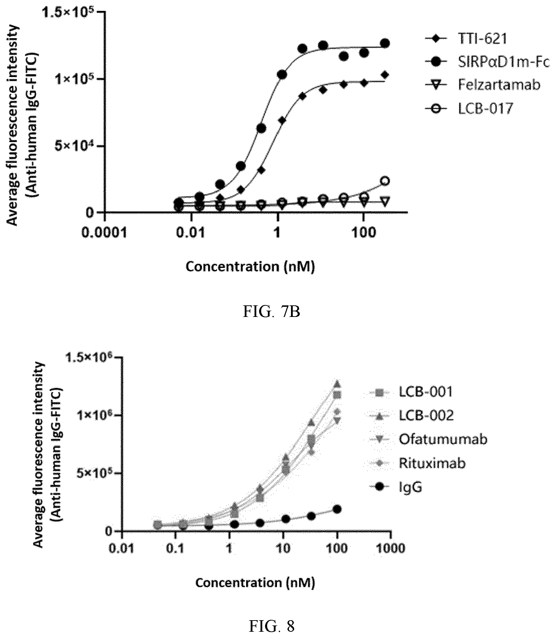

For example, as shown in FIG. 7B, the high-affinity SIRPα D1m-Fc fusion protein, the anti-CD47 fusion protein TTI-621, and the recombinant protein LCB-017 can all bind to non-target cells of interest CHO-K1 cells. Specifically, the affinity of the recombinant protein LCB-017 is significantly weaker than that of the anti-CD47 fusion protein TTI-621 (SIRPα D1-Fc fusion protein).

Other bispecific recombinant proteins of the present disclosure whose second functional antigen is CD47 can also be observed for their low binding affinity or no binding to non-target cells of interest.

The above experimental data prove that the recombinant protein of the present disclosure can specifically target the CD47 antigen of tumor cells at the cellular level, and the binding affinity to CD47 is significantly weaker than that of the SIRPα D1-Fc fusion protein to CD47; the bispecific recombinant protein of the present disclosure can unexpectedly significantly reduce or avoid side effects including non-tumor target cell killings, such as hemagglutination, anemia, and thrombocytopenia which are produced by anti-CD47 antibody or SIRPα D1-Fc fusion protein treatment.

### Surface Plasmon Resonance Analysis (hereafter "SPR Analysis") on affinity of the bispecific recombinant protein to the target CD20 or CD47:

The following method takes LCB-002 as an example, and is suitable for the assay of the recombinant protein whose first functional antigen is CD20 and whose second functional antigen is CD47.

Biacore T200 (GE Healthcare) was used for SPR Analysis. An anti-human IgG (Fc) antibody (Human Antibody Capture Kit, GE Healthcare) was immobilized on a CM5 sensor chip. 5 µg/mL of human CD20-Fc or human CD47-Fc chimeric proteins diluted with HBS-EP (GE Healthcare) were each allowed to immobilize, and the amount of capture was measured. Subsequently, the complete recombinant protein LCB-002, anti-CD20 antibody Ofatumumab, and anti-CD47 fusion protein TTI-621 were diluted to 50 nM with HBS-EP, and their binding capacities with human CD20-Fc chimeric protein or human CD47-Fc chimeric protein were measured respectively. Then, HBS-EP+buffer was added at a flow rate of 50 µL/min for 5 minutes, and the dissociation between the recombinant protein LCB-002, anti-CD20 antibody Ofatumumab, anti-CD47 fusion protein TTI-621 and human CD20-Fc chimeric protein or human CD47-Fc chimeric protein was measured. The bivalent analyte model and Rmax were analyzed by Fit local to calculate the association rate constant (ka) and the dissociation rate constant (kd), and the association-dissociation constant (KD) was calculated by dividing kd by ka. The Kd values of recombinant protein LCB-002, anti-CD20 antibody Ofatumumab, anti-CD47 fusion protein TTI-621 to human CD20-Fc chimeric protein or human CD47-Fc chimeric protein are shown in Table 3:

**Table 3: SPR Analysis on the association-dissociation constants of recombinant proteins or control samples to the first or second functional antigen**

| Sample name | Kd (hCD20) | Kd (hCD47) |
|---|---|---|
| LCB-002 | 3.24 nM | 52.8 nM |
| Ofatumumab | 1.98 nM | - |
| TTI-621 | - | 37.3 nM |

Ofatumumab specifically targets the human CD20 protein, and therefore does not bind to the human CD47-Fc chimeric protein, while the anti-CD47 fusion protein TTI-621 specifically targets the CD47 protein, and therefore does not bind to the human CD20-Fc chimeric protein.

The results show that at the protein level, the recombinant protein LCB-002 can bind to human CD47 protein or human CD20 protein, respectively, and its affinity does not change significantly compared with the anti-CD20 antibody Ofatumumab or the anti-CD47 fusion protein TTI-621.

The above experimental data prove that the recombinant protein of the present disclosure can specifically target the CD20 antigen and CD47 antigen of tumor cells at the protein level, and the binding affinity to CD47 or CD20 is comparable to that of anti-CD20 antibody Ofatumumab or anti-CD47 fusion protein TTI-621.

### Determination of bispecific binding to the targets CD20 and CD47 by flow cytometry:

The binding affinity of the bispecific recombinant protein to the targets CD20 and CD47 was determined by flow cytometry. The following method takes LCB-001 or LCB-002 as an example, and is suitable for the assay of the recombinant protein whose first functional antigen is CD20 and whose second functional antigen is CD47.

Well-grown Raji cells (human B-cell lymphoma) (Cell Bank of Chinese Academy of Sciences, Shanghai) (CD20+/CD47+, target cells of interest) were collected and counted, centrifuged and resuspended in PBS+2% FBS to a concentration of 3×10⁶ cells/mL. 100 µL of the cell suspension was aliquoted to each well of a 96-well U-shaped plate (Art. No. 3799, Corning), and 8 dilution gradients of LCB-001, LCB-002, Ofatumumab, Rituximab, IgG (3-fold serial dilutions starting from 100 nM with 8 concentrations in total) were added respectively, and the plate was incubated at 4°C for 1 hour; after rinsing with PBS+2% FBS, goat anti-human IgGFc-FITC (F9512-2ML, Sigma) was added and the plate was incubated at 4°C for 1 hour; after rinsing and resuspension with PBS+2% FBS, the fluorescence value was determined by flow cytometry (BD).

As shown in FIG. 8, since both CD20 and CD47 antigens are expressed on the surface of Raji cells, both anti-CD20 antibodies Ofatumumab and Rituximab can specifically bind to Raji cells, while the recombinant proteins LCB-001 and LCB-002 can also bind to Raji cells, and their binding abilities are similar to Ofatumumab and Rituximab.

### 2. Assay of competitive binding activity to the target

The following method takes LCB-001, LCB-005, LCB-006, and LCB-017 as examples, and is suitable for the bispecific recombinant protein whose second functional antigen is CD47.

### Determination of competitive binding activity of LCB-001 by flow cytometry:

The competitive binding activity of the bispecific recombinant proteins LCB-001 and LCB-002 and the potential impurity control samples LCB-001-R and LCB-002-R, competing with FITC-labeled high-affinity SIRPα D1m-Fc (SIRPα(CV1)-Fc-FITC) for binding to CD47 on the cell surface was determined by flow cytometry. The following method takes LCB-001 or LCB-002 as an example, and is suitable for the assay of the recombinant protein whose first functional antigen is CD20 and whose second functional antigen is CD47.

Well-grown Raji cells (human B-cell lymphoma) (Cell Bank of Chinese Academy of Sciences, Shanghai) were collected and counted, centrifuged and resuspended in PBS+2% FBS to a concentration of 3×10⁶ cells/mL. 100 µL of the cell suspension was aliquoted to each well of a 96-well U-shaped plate (Art. No. 3799, Corning), and serially diluted LCB-001 (5-fold dilutions starting from a final concentration of 200,000 ng/mL with 8 concentration points in total) was added. The final concentration of FITC-labeled high-affinity SIRPα D1m-Fc (SIRPα(CV1)-Fc-FITC) was 4 nM. LCB-001, SIRPα(CV1)-Fc-FITC and Raji cells were co-incubated for 1 hour, and the supernatant was discarded by centrifugation. The cells were resuspended with DPBS+2% FBS, and then determined by flow cytometry.

The results show that anti-CD47 antibody Magrolimab, anti-CD47 fusion protein TTI-621, recombinant proteins LCB-001 and LCB-002 can compete in varying degrees with FITC-labeled high-affinity SIRPα D1m-Fc for binding to the CD47 antigen on Raji cells, and can exert competitive binding activity; potential impurities LCB-001-R and LCB-002-R cannot compete with FITC-labeled high-affinity SIRPα D1m-Fc for binding to the CD47 antigen, and cannot exert competitive binding activity.

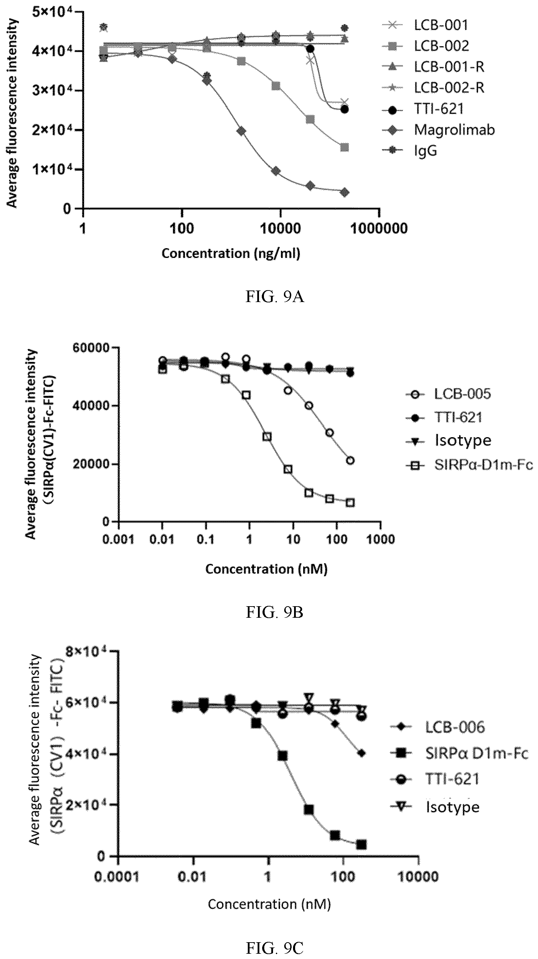

For example, as shown in FIG. 9A, anti-CD47 antibody Magrolimab, anti-CD47 fusion protein TTI-621, recombinant proteins LCB-001 and LCB-002 can compete in varying degrees with FITC-labeled high-affinity SIRPα D1m-Fc for binding to the CD47 antigen, and can exert competitive binding activity, and the competitive binding activity of the recombinant protein LCB-002 is non-obviously better than that of anti-CD47 fusion protein TTI-621, while the competitive binding activity of the recombinant protein LCB-001 is comparable to that of anti-CD47 fusion protein TTI-621. The results show that the recombinant protein using a suitable linker to connect the first functional structural fragment and the second functional structural fragment can non-obviously improve the competitive binding ability of the second functional structural fragment to the second functional antigen. At the same time, the potentially risky impurity proteins LCB-001-R and LCB-002-R cannot compete with FITC-labeled high-affinity SIRPα D1m-Fc for binding to the CD47 antigen. According to the results, the potentially risky impurities, which are generated during the preparation of recombinant proteins and are difficult to be completely removed, have significantly reduced the competitive binding activity to the second functional antigen. It can be inferred that the binding capability of the potentially risky impurities to the second functional antigen on non-target cells of interest is very weak, which greatly reduces the immune-related toxic side effects and has an excellent safety profile.

### Determination of competitive binding activity of LCB-005 by flow cytometry:

The competitive binding activity of the bispecific recombinant protein LCB-005, competing with FITC-labeled high-affinity SIRPα D1m-Fc (SIRPα(CV1)-Fc-FITC) for binding to CD47 on the cell surface was determined by flow cytometry. The following method takes LCB-005 as an example, and is suitable for the assay of the recombinant protein whose first functional antigen is EpCAM and whose second functional antigen is CD47.

Well-grown target cells of interest CAPAN-2 cells (human pancreatic cancer cells) (EpCAM+/CD47+) (purchased from Nanjing Kebai) were rinsed and digested, counted, centrifuged and resuspended with DPBS+2% FBS to a concentration of 3 × 10⁶ cells/mL. 100 µL of the cell suspension was aliquoted to each well of a 96-well U-shaped plate (Art. No. 3799, Corning), and the LCB-005 was serially diluted (3-fold dilutions starting from a final concentration of 200 nM with 8 concentration points in total). The final concentration of FITC-labeled high-affinity SIRPα D1m-Fc (SIRPα(CV1)-Fc-FITC) was 4 nM. LCB-006, SIRPα D1m-Fc, TTI-621, SIRPα(CV1)-Fc-FITC, and CAPAN-2 cells were co-incubated for 1 hour, and the supernatant was discarded by centrifugation. The cells were resuspended with DPBS+2% FBS, and then determined by flow cytometry.

The results show that, compared with the anti-CD47 fusion protein TTI-621, which has no obvious competitive binding activity on CAPAN-2 cells, both the bispecific recombinant protein LCB-005 and the high-affinity SIRPα D1m-Fc can compete in varying degrees with FITC-labeled high-affinity SIRPα D1m-Fc (SIRPα(CV1)-Fc-FITC) for binding to the CD47 antigen on CAPAN-2 cells, and can exert competitive binding activity. The competitive binding capability of the bispecific recombinant protein LCB-005 to CAPAN-2 is significantly better than that of the anti-CD47 fusion protein TTI-621.

For example, as shown in FIG. 9B, both the bispecific recombinant protein LCB-005 and the high-affinity SIRPα D1m-Fc can compete in varying degrees with FITC-labeled high-affinity SIRPα D 1m-Fc (SIRPα(CV1)-Fc-FITC) for binding to the CD47 antigen, and can exert competitive binding activity, while the competitive binding activity of anti-CD47 fusion protein TTI-621 is not observed. It can be seen that the competitive binding activity of the bispecific recombinant protein LCB-005 is non-obviously better than that of anti-CD47 fusion protein TTI-621. The results show that the bispecific recombinant protein of the present disclosure using a suitable linker to connect the first functional structural fragment and the second functional structural fragment can non-obviously improve the competitive binding ability of the second functional structural fragment to the second functional antigen.

### Determination of competitive binding activity of LCB-006 by flow cytometry:

The competitive binding activity of the bispecific recombinant protein LCB-006, competing with FITC-labeled high-affinity SIRPα D1m-Fc (SIRPα(CV1)-Fc-FITC) for binding to CD47 on the cell surface was determined by flow cytometry. The following method takes LCB-006 as an example, which is suitable for the assay of the recombinant protein whose first functional antigen is CD24 and whose second functional antigen is CD47.

Well-grown target cells of interest MCF-7 cells (human breast cancer cells) (CD24+/CD47+) (purchased from Nanjing Kebai) were rinsed and digested, counted, centrifuged and resuspended with DPBS+2% FBS to a concentration of 3×10⁶ cells/mL. 100 µL of the cell suspension was aliquoted to each well of a 96-well U-shaped plate (Art. No. 3799, Corning), and the LCB-006 was serially diluted (3-fold dilutions starting from a final concentration of 200 nM with 8 concentration points in total). The final concentration of FITC-labeled high-affinity SIRPα D1m-Fc (SIRPα(CV1)-Fc-FITC) was 4 nM. LCB-006, SIRPα D1m-Fc, TTI-621, SIRPα(CV1)-Fc-FITC and MCF-7 cells were co-incubated for 1 hour, and the supernatant was discarded by centrifugation. The cells were resuspended with DPBS+2% FBS, and then determined by flow cytometry.

The results show that, compared with the anti-CD47 fusion protein TTI-621, which has no obvious competitive binding activity on MCF-7 cells, both the bispecific recombinant protein LCB-006 and the high-affinity SIRPα D1m-Fc can compete in varying degrees with FITC-labeled high-affinity SIRPα D1m-Fc (SIRPα(CV1)-Fc-FITC) for binding to the CD47 antigen on MCF-7 cells, and can exert competitive binding activity. The competitive binding activity of the bispecific recombinant protein LCB-006 to MCF-7 is significantly better than that of the anti-CD47 fusion protein TTI-621.

For example, as shown in FIG. 9C, both the bispecific recombinant protein LCB-006 and the high-affinity SIRPα D1m-Fc can compete in varying degrees with FITC-labeled high-affinity SIRPα D 1m-Fc (SIRPα(CV1)-Fc-FITC) for binding to the CD47 antigen, and can exert competitive binding activity, while the competitive binding activity of anti-CD47 fusion protein TTI-621 is not observed. It can be seen that the competitive binding activity of the bispecific recombinant protein LCB-006 is non-obviously better than that of anti-CD47 fusion protein TTI-621. The results show that the bispecific recombinant protein of the present disclosure using a suitable linker to connect the first functional structural fragment and the second functional structural fragment can non-obviously improve the competitive binding ability of the second functional structural fragment to the second functional antigen.

### Determination of competitive binding activity of LCB-017 by flow cytometry:

The competitive binding activity of the bispecific recombinant protein LCB-017, competing with FITC-labeled high-affinity SIRPα D1m-Fc (SIRPα(CV1)-Fc-FITC) for binding to CD47 on the cell surface was determined by flow cytometry. The following method takes LCB-017 as an example, and is suitable for the assay of the recombinant protein whose first functional antigen is CD38 and whose second functional antigen is CD47.

Well-grown Raji cells (human B-cell lymphoma) (Cell Bank of Chinese Academy of Sciences, Shanghai) (CD38+/CD47+) were collected, counted and centrifuged, and the cells were resuspended with DPBS+2% FBS to a concentration of 3×10⁶ cells/mL. 100 µL of a cell suspension was aliquoted to each well of a 96-well U-shaped plate (Art. No. 3799, Corning), and the LCB-017 was serially diluted (3-fold dilutions starting from a final concentration of 200 nM with 7 concentration points in total). The final concentration of FITC-labeled high-affinity SIRPα D1m-Fc (SIRPα(CV1)-Fc-FITC) was 4 nM. LCB-017, SIRPα D1m-Fc, TTI-621, SIRPα(CV1)-Fc-FITC and Raji cells were co-incubated for 1 hour, and the supernatant was discarded by centrifugation. The cells were resuspended with DPBS+2% FBS, and then determined by flow cytometry.

The results show that anti-CD47 fusion protein TTI-621, the bispecific recombinant protein LCB-017 and high-affinity SIRPα D1m-Fc can all compete in varying degrees with FITC-labeled high-affinity SIRPα D1m-Fc for binding to the CD47 antigen on Raji cells, and exert competitive binding activity. The competitive binding activity of the bispecific recombinant protein LCB-017 to MCF-7 is significantly better than that of the anti-CD47 fusion protein TTI-621.

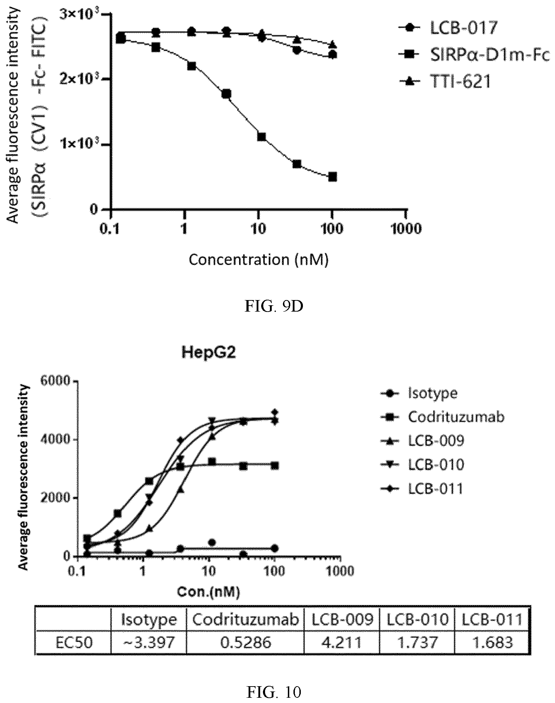

For example, as shown in FIG. 9D, anti-CD47 fusion protein TTI-621, the bispecific recombinant protein LCB-017, and high-affinity SIRPα D1m-Fc can all compete in varying degrees with FITC-labeled high-affinity SIRPα D1m-Fc for binding to the CD47 antigen on Raji cells, and exert competitive binding activity. It can be seen that the competitive binding activity of the bispecific recombinant protein LCB-017 (IC50=27.98) is non-obviously better than that of the anti-CD47 fusion protein TTI-621 (IC50=1067). The results show that the bispecific recombinant protein of the present disclosure using a suitable linker to connect the first functional structural fragment and the second functional structural fragment can non-obviously improve the competitive binding ability of the second functional structural fragment to the second functional antigen.

To sum up (FIG. 9A-FIG. 9D), the bispecific recombinant protein of the present disclosure using a suitable linker to connect the first functional structural fragment and the second functional structural fragment can non-obviously improve the competitive binding ability of the second functional structural fragment to the second functional antigen. The results are also observed in other bispecific recombinant proteins whose second functional antigen is CD47 on target cells of interest with positive antigen of interest, which are consistent with the above data.

### Embodiment 4: Binding activity assay of GPC3-targeted bispecific recombinant proteins to liver cancer cell lines highly expressed with GPC3 (double-positive expressing cells, target cells of interest)

The binding affinity of the GPC3-targeted bispecific recombinant protein to the liver cancer cell line highly expressed with the target GPC3 was determined by flow cytometry. The following method takes LCB-009, LCB-010, and LCB-011 as examples, and is suitable for the assay of the bispecific recombinant protein whose first functional binding fragment binds to GPC3 antigen and whose second functional binding fragment is IFN-α 2b or its low-affinity mutant.

The cells for the assay were HepG2 cells (Nanjing Kebai Biotechnology Co., Ltd.) and HuH-7 (Cell Bank of Chinese Academy of Sciences, Shanghai). Well-grown cells were collected and counted, centrifuged and resuspended in FACS buffer (PBS+2% FBS) to a concentration of 1×10⁶ cells/mL. 100 µL of the cell suspension was aliquoted to each well of a 96-well U-shaped plate (Art. No. 3799, Corning), and 7 dilution gradients of bispecific recombinant proteins or control proteins (3-fold serial dilutions starting from 100 nM with 7 concentrations in total) were added respectively, and the plate was incubated at 4°C for 1 hour; after rinsing with FACS buffer, goat anti-human IgG (Alexa Fluor488 goat anti-human IgG (H+L), Invitrogen) was added and the plate was incubated at 4°C for 1 hour; after rinsing and resuspension with FACS buffer, the fluorescence value was determined by flow cytometer (Attune Nxt, Invitrogen).

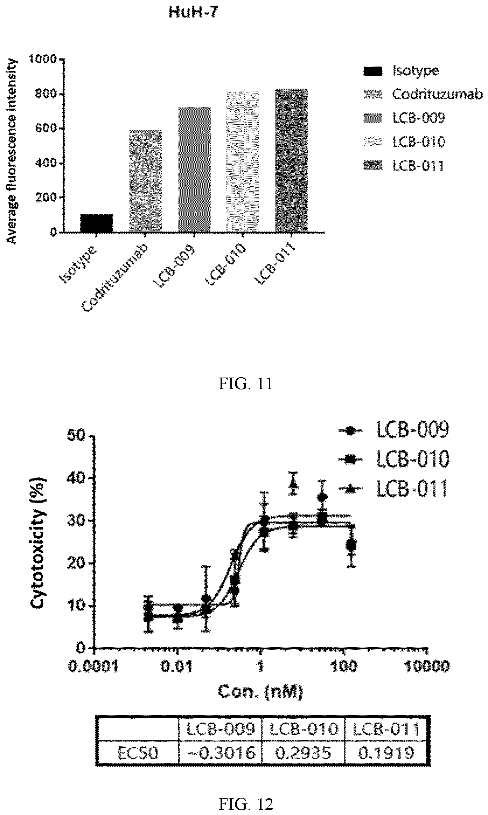

The results are shown in FIG. 10 and FIG. 11. The bispecific recombinant proteins LCB-009, LCB-010, and LCB-011 all have a certain degree of binding activity with the target cells of interest HepG2 and HuH-7 cells that express GPC3 and IFNα receptors simultaneously, and the binding of the bispecific recombinant protein to HepG2 and HuH-7 cells has a higher maximum average fluorescence intensity than that of anti-GPC3 mAb (control sample Codrituzumab). At the same time, as shown in FIG. 10 and FIG. 11, the length of the linker has a weak effect on the binding activity of the bispecific recombinant protein to the target cells of interest. When the linker is short (for example, the linker sequence is one GGGGS), the binding activity (EC50) of bispecific recombinant proteins (such as LCB-009) to target cells of interest is relatively low.

### Embodiment 5: Activity assay of antibody-dependent cell-mediated cytotoxicity (ADCC) of GPC3-targeted bispecific recombinant protein

The ADCC activity of the bispecific recombinant protein on the hepatoma cell line highly expressed with the target GPC3 was determined by lactate dehydrogenase (LDH) method. The following method takes LCB-009, LCB-010, and LCB-011 as examples, and is suitable for the bispecific recombinant protein whose first functional binding fragment binds to GPC3 antigen and whose second functional binding fragment is IFN-α 2b or its low-affinity mutant.

Human peripheral blood mononuclear cells (PBMC) were used as effector cells, the target cells of interest were HepG2 liver cancer cell line highly expressed with GPC3, and the LDH assay kit was CytoTox-ONETM-Homogeneous Membrance Integrity Assay, Promega, G7892. Target cells of interest and effector cells were plated at a ratio of 1:20, then bispecific recombinant proteins or control proteins (5-fold serial dilutions starting from 150 nM with 8 concentrations in total) were added, and the plate was incubated at 37°C for 4 hours. After incubation at 37°C for 3.5 hours, lysis reagent was added to the control group, and the cells were observed under a microscope. After the cells were completely lysed, they were centrifuged at 10,000 rpm for 5 minutes. The supernatant was transferred to a 96-well black-bottomed transparent plate (Corning, 3904), and incubated with the reaction substrate at 37°C for 30 minutes. The termination solution was then added, and the plate was shaked in the dark for 3 to 5 minutes. The signal value was detected by the microplate reader (SpectraMax M2), and the detailed steps were referred from the LDH assay kit instructions.

As shown in FIG. 12, the bispecific recombinant protein (such as LCB-009, LCB-010, LCB-011) retains ADCC activity, and the ADCC activity of the bispecific recombinant protein is comparable to that of anti-GPC3 mAb (control sample Codrituzumab) (EC50=0.53).

### Embodiment 6: Assay of proliferation inhibition activity of GPC3-targeted bispecific recombinant protein

The proliferation inhibition activities of the GPC3-targeted bispecific recombinant protein on different tumor cell lines were determined by cell titer glo kit (Promega, Cat: G7558). The following method takes LCB-009, LCB-010, and LCB-011 as examples, and is suitable for the assay of the bispecific recombinant protein whose first functional binding fragment binds to GPC3 antigen and whose second functional binding fragment is IFNα 2b or its low-affinity mutant.

GPC3-positive cell line HuH-7 or GPC3-negative tumor cell line U266 (purchased from Nanjing Kebai Biotechnology Co., Ltd.) and GPC3-negative tumor cell line SW480 (purchased from Nanjing Kebai Biotechnology Co., Ltd.) were plated in a 96-well black-bottomed transparent plate (Corning, 3904), then bispecific recombinant proteins or control proteins (10-fold serial dilutions starting from 52 nM with 6 points in total; or 5-fold serial dilutions starting from 10 nM with 8 points in total) were added, and the plate was incubated in a carbon dioxide incubator at 37°C for 3 days. Cell titer glo was added, and the signal value was detected by Multomode Plate Reader (PerkinElmer, Envision2105).

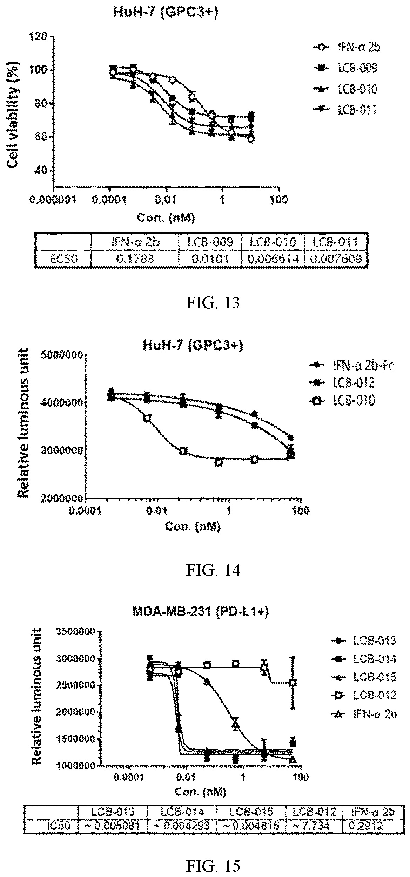

The results show that the proliferation inhibition activity (IC50) of the bispecific recombinant protein (such as LCB-009, LCB-010, LCB-011) on GPC3-positive (GPC3+) target cells of interest HuH-7 is higher than that of the control IFN-α 2b (as shown in FIG. 13), and is higher than that of the anti-GPC3 mAb (i.e. the control sample Codrituzumab, the proliferation inhibition effect of this antibody on HuH-7 is not observed) as well. As shown in FIG. 14, the proliferation inhibition activities of the bispecific recombinant protein LCB-012 lacking the function of targeting GPC3 and the Fc fusion protein of IFN-α2b (IFN-α2b-Fc) on HuH-7 (GPC3-positive target cells of interest) are significantly lower than that of the bispecific recombinant protein LCB-010 with the function of targeting GPC3, indicating that the binding of the bispecific recombinant protein with the function of targeting GPC3 to GPC3 on target cells of interest can significantly enhance the proliferation inhibition activity of IFN-α 2b, but IFN-α2b of the bispecific recombinant protein without the effect of targeting the target cells of interest has a low proliferation inhibition activity. It reveals that the bispecific recombinant protein of the present disclosure has a strong proliferation inhibition effect only on the target cells of interest with the antigen of interest, but has a weak effect on or does not bind to the non-target cells of interest without the antigen of interest, indicating that the bispecific recombinant protein of the present disclosure has a high safety profile.

In addition, as shown in Table 4, compared with IFN-α 2b, the bispecific recombinant proteins with different linkers (taking LCB-009, LCB-010, LCB-011 as examples) have stronger proliferation inhibition activities on GPC3-positive (GPC3+) target cells of interest (taking HuH-7 as an example), and the proliferation inhibition activities of the bispecific recombinant proteins with different linkers differ slightly. The bispecific recombinant protein containing one GGGGS as the linker sequence (i.e. the bispecific recombinant protein connected by a shorter linker) (taking LCB-009 as an example) has relatively low activity (as shown in FIG. 13 and Table 4), which is consistent with the results of binding activities of the recombinant proteins with different linkers as shown in FIG. 11. But in GPC3-negative (GPC3-) non-target cells of interest U266 and SW480 cell lines, the proliferation inhibition activities of the bispecific recombinant proteins (taking LCB-009, LCB-010, LCB-011 as examples) are significantly lower than that of IFN-α2b (that is, the relative activity of the bispecific recombinant protein is much less than 1), which indicates that in cells that do not express GPC3 (i.e. non-target cells of interest that do not express the antigen of interest), the proliferation inhibition activity of the bispecific recombinant protein is very low, suggesting a high safety profile. At the same time, as shown in Table 4, the proliferation inhibition activity of the bispecific recombinant protein on target cells of interest is at least 700 times higher than that of the bispecific recombinant protein on non-target cells of interest.

**Table 4: The relative proliferation inhibition activities of bispecific recombinant proteins with different linkers**

| Sample | Relative activity (IC50 IFN-α 2b/bispecific recombinant protein) | | | Relative activity ratio (target cell of interest /non-target cell of interest) | |
|---|---|---|---|---|---|
| | HuH-7 (GPC3+) | U266 (GPC3-) | SW480 (GPC3-) | HuH-7(GPC3+)/ U266(GPC3-) | HuH-7(GPC3+)/ SW480(GPC3-) |
| LCB-009 | 17.80 | 0.008 | 0.023 | 2225.000 | 773.913 |
| LCB-010 | 27.00 | 0.034 | 0.017 | 794.118 | 1588.235 |
| LCB-011 | 23.40 | 0.004 | 0.030 | 5850.000 | 780.000 |

### Embodiment 7: Assay of proliferation inhibition activity of PD-L1-targeted bispecific recombinant protein

The proliferation inhibition activities of the PD-L1-targeted bispecific recombinant protein on different tumor cell lines were determined by cell titer glo kit (Promega, Cat: G7558). The following method takes LCB-013, LCB-014, and LCB-015 as examples, and is suitable for the bispecific recombinant protein whose first functional binding fragment binds to PD-L1 antigen and whose second functional binding fragment is IFN-α 2b or its low-affinity mutant.

PD-L1 positive cell line MDA-MB-231 (purchased from Nanjing Kebai Biotechnology Co., Ltd.) was plated in a 96-well black-bottomed transparent plate (Corning, 3904), then bispecific recombinant proteins or control proteins (10-fold serial dilutions starting from 52 nM with 6 concentration points in total) were added, and the plate was incubated in a carbon dioxide incubator at 37°C for 3 days. Cell titer glo was added, and the signal value was detected by Multomode Plate Reader (PerkinElmer, Envision2105).

As shown in FIG. 15, for PD-L1-positive (PD-L1+) target cells of interest MDA-MB-231, PD-L1-targeted bispecific recombinant proteins (LCB-013, LCB-014, LCB-015) all have proliferation inhibition activity, and the activity is significantly higher than the control bispecific recombinant protein LCB-012 without the function of targeting PD-L1 and IFN-α 2b, indicating that targeting PD-L1 can significantly enhance the proliferation inhibition activity of IFN-α2b. It is shown that the bispecific recombinant protein with the function of targeting PD-L1 binding to PD-L1 on the target cell of interest MDA-MB-231 can significantly enhance the proliferation inhibition activity of IFN-α2b, but the proliferation inhibition activity of IFN-α2b of the bispecific recombinant protein without the function of targeting the target cell of interest is relatively low. It reveals that the bispecific recombinant protein of the present disclosure has a strong proliferation inhibition effect only on the target cells of interest with the antigen of interest, but has a weak effect on or does not bind to the non-target cells of interest that do not express the antigen of interest, indicating that the bispecific recombinant protein of the present disclosure has a high safety profile.

PD-L1-positive target cells of interest MDA-MB-231 were plated in a 96-well black-bottomed transparent plate (Corning, 3904), then 200 nM of PD-L1 antibody Atezolizumab or isotype control was added, and the plate was incubated at 37°C for 30 minutes. Bispecific recombinant proteins or control proteins (6-fold dilutions starting from a concentration of 20 nM with 6 concentration points in total) were added, and the plate was incubated in a carbon dioxide incubator at 37°C for 3 days. Cell titer glo was added, and the signal value was detected by Multomode Plate Reader (PerkinElmer, Envision2105).

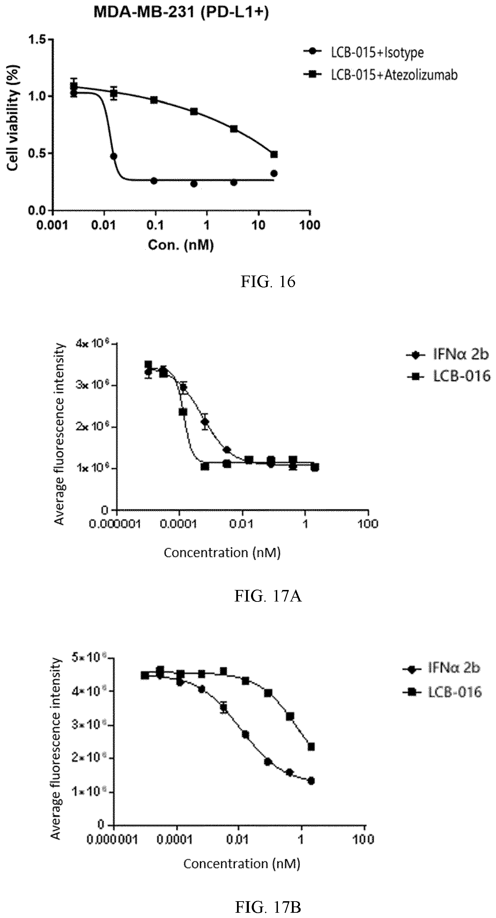

As shown in FIG. 16, after Atezolizumab was added to block the binding site of PD-L1 antigen and the antibody, the proliferation inhibition activity of LCB-015 was significantly reduced. The results prove that blocking the binding function of the antigen of interest on the cells to be tested results in a significant decrease in the proliferation inhibition activity of the bispecific recombinant protein on the cells to be tested, which demonstrates from another perspective that the bispecific recombinant protein of the present disclosure has a weak effect on or does not bind to non-target cells of interest without the ability to bind to the antigen of interest, indicating that the bispecific recombinant protein of the present disclosure has a high safety profile.

### Embodiment 8: Assay of proliferation inhibition activity of CD38-targeted bispecific recombinant protein

The proliferation inhibition activities of the CD38-targeted bispecific recombinant protein on different tumor cell lines were determined by cell titer glo kit (Promega, Cat: G7558). The following method takes LCB-016 as an example, and is suitable for the assay of the bispecific recombinant protein whose first functional binding fragment binds to CD38 antigen and whose second functional binding fragment is IFNα2b.

CD38-positive cell line Daudi or CD38-negative tumor cell line SK-BR-3 (purchased from Nanjing Kebai Biotechnology Co., Ltd.) was plated in a 96-well black-bottomed transparent plate (Corning, 3904), then bispecific recombinant proteins or control proteins (5-fold serial dilutions starting from 2 nM with 9 points in total) were added, and the plate was incubated in a carbon dioxide incubator at 37°C for 3 days. Cell titer glo was added, and the signal value was detected by Multomode Plate Reader (PerkinElmer, Envision2105).

The results show that the proliferation inhibition activity (IC50) of the bispecific recombinant protein (such as LCB-016) on the CD38-positive (CD38+) target cell of interest Daudi is non-obviously stronger than that of the control IFN-α 2b (as shown in FIG. 17A), relatively speaking, the proliferation inhibition activity (IC50) of the bispecific recombinant protein (such as LCB-016) on the CD38-negative (CD38-) non-target cell of interest SK-BR3 is non-obviously weaker than that of the control IFN-α 2b (as shown in FIG. 17B). That is, while the proliferation inhibition activity of IFNα 2b in the bispecific recombinant protein whose second functional binding fragment is IFNα 2b on the target cells of interest with the antigen of interest is enhanced, the proliferation inhibition effect of IFNα 2b in the bispecific recombinant protein on the non-target cells of interest without the antigen of interest is significantly reduced. It is revealed that the bispecific recombinant protein of the present disclosure has a strong proliferation inhibition effect only on the target cells of interest with the antigen of interest, but has a weak effect on or does not bind to the non-target cells of interest without the antigen of interest, indicating that the bispecific recombinant protein of the present disclosure has a high safety profile.

As shown in FIG. 17A, the CD38-targeted bispecific recombinant protein (LCB-016) has proliferation inhibition activity on the CD38-positive (CD38+) target cell of interest Daudi, and the activity is significantly higher than that of IFN-α 2b without the function of targeting CD38, indicating that targeting CD38 can significantly enhance the proliferation inhibition activity of IFN-α 2b. It is shown that the bispecific recombinant protein with the function of targeting CD38 binding to CD38 on the target cell of interest Daudi can significantly enhance the proliferation inhibition activity of IFN-α 2b. As shown in FIG. 17B, the proliferation inhibition activity of IFN-α 2b lacking the function of targeting CD38 on SK-BR3 (CD38-negative non-target cell of interest) is significantly stronger than that of the bispecific recombinant protein LCB-016 with the function of targeting CD38, indicating that IFN-α 2b of the bispecific recombinant protein exerts a low proliferation inhibition activity on non-target cells of interest without the antigen of interest. The relative activity of LCB-016 to IFN-α 2b on the target cell of interest Daudi is at least 200 times higher than that on the non-target cell of interest SK-BR3.

### Embodiment 9: Assay of proliferation inhibition activity of the bispecific recombinant protein containing IFN-α 2b low-affinity mutant

Considering the difference between the human body-tolerated dose of IFN-α 2b and the effective dose of conventional antibodies, in order to better match the effect of the antigen of interest-targeted antigen-binding fragment and IFN-α 2b, and achieve the effect of high efficiency and low toxicity, the present disclosure also designed a series of bispecific recombinant proteins containing IFN-α 2b low-affinity mutants. This embodiment takes the mutation design based on LCB-010 as an example to design the bispecific recombinant proteins containing IFN-α 2b low-affinity mutants, and to determine the proliferation inhibition activity of the bispecific recombinant proteins containing different IFN-α 2b low-affinity mutants. The experimental method is the same as Embodiment 7.

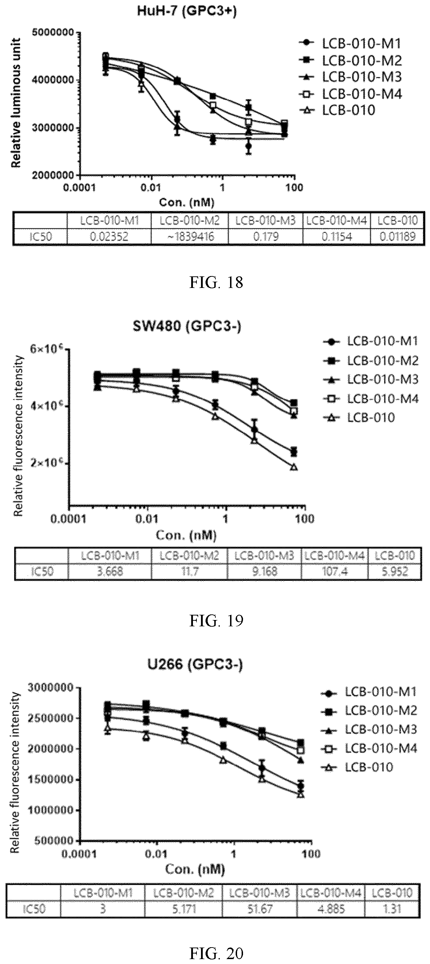

As shown in FIG. 18-FIG. 20, the results show that the proliferation inhibition activities of the bispecific recombinant proteins with IFN-α 2b low-affinity mutants LCB-010-M2, LCB-010-M3 and LCB-010-M4 on GPC3-positive target cells of interest (HuH-7) or GPC3-negative non-target cells of interest (U266, SW480) are all lower than that of LCB-010. For example, the proliferation inhibition activity IC50 of LCB-010-M3 (A145G mutation) on HuH-7 (GPC3-positive cell line, target cell of interest) is comparable to that of IFN-α 2b (IC50_{IFN-α 2b}=0.1793, IC50_{LCB-010-M3}=0.179), but the relative proliferation inhibition activity (IC50 IFN-α 2b/bispecific recombinant protein) of LCB-010-M3 on GPC3-negative non-target cell of interest (U266) is weaker, e.g., the relative proliferation inhibition activity (IC50 IFN-α 2b/bispecific recombinant protein) of LCB-010-M3 on U266 is 0.000615, which is significantly lower than that of IFN-α 2b and lower than that of LCB-010, and LCB-010-M4 (R149A mutation) also has similar results.

The above results and FIG. 19-FIG. 21 show that the activity of the GPC3-targeted bispecific recombinant protein containing the IFN-α 2b low-affinity mutant in the target cell line with positive antigen of interest is comparable to that of IFN-α 2b, but the activity decreases in the non-target cell line that does not express the antigen of interest, suggesting a higher safety profile of the bispecific recombinant protein containing the IFN-α 2b low-affinity mutant of the present disclosure.

In summary, the degree of decrease in the proliferation inhibition activity of the bispecific recombinant protein containing the IFN-α 2b low-affinity mutant of the present disclosure relative to the bispecific recombinant protein containing wild-type IFN-α 2b on non-target cells of interest with negative antigen of interest, is comparable to or more significant than the degree of decrease in the proliferation inhibition activity of the bispecific recombinant protein containing the IFN-α 2b low-affinity mutant on non-target cells of interest with negative antigen of interest relative to that on target cells of interest with positive antigen of interest.

### Embodiment 10: Assay of proliferation inhibition activity of potentially risky impurities of the bispecific recombinant protein on non-target cell of interest.

In order to reduce the potential safety risk of impurities in the future preparation process of the bispecific recombinant protein, the present disclosure takes LCB-010 as an example to analyze the proliferation inhibition of potentially risky impurities (chain B homodimer) on non-target cells of interest.

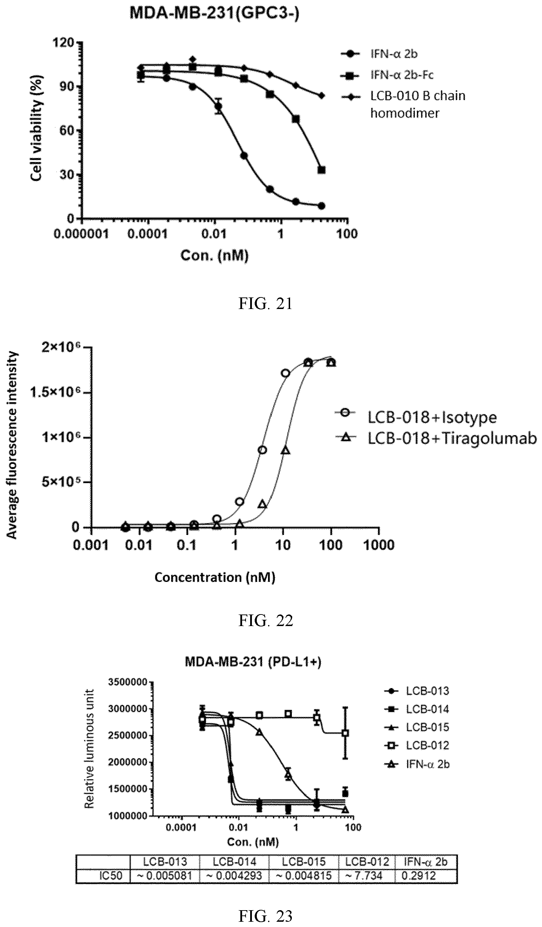

In the secondary purification process of LCB-010 (as described in Embodiment 2), the chain B homodimer of LCB-010 (FIG. 3, the molecular weight of the homodimer is about 140kD) was isolated, and the proliferation inhibition activities of the potentially risky impurity and IFN-α 2b-Fc (the potentially risky impurity of the bi-clonal antibody structure shown in FIG. 3A of CN108864290A, the right arm homodimer with D1 replaced by IFN-α 2b) on non-target cells of interest (GPC3-negative cells) MDA-MB-231 were determined. The results are shown in FIG. 21 that, at the concentration of 16.7 nM, the proliferation inhibition rate of IFN-α 2b on MDA-MB-231 (GPC3-negative cell, non-target cell of interest) is 91.3%, the inhibition rate of IFN-α 2b-Fc on MDA-MB-231 (GPC3-negative cell, non-target cell of interest) is 66.8% (reduced by about 24.5% compared with IFN-α 2b), while the proliferation inhibition rate of the chain B homodimer of LCB-010 on MDA-MB-231 (GPC3-negative cell, non-target cell of interest) is only 16.2% (reduced by about 75.1% compared with IFN-α 2b, and reduced by about 50% compared with IFN-α 2b-Fc). To sum up, the potentially risky impurities only have a very weak effect on non-target cells of interest, that is, the potential safety risks or potential toxic side effects are very low.

### Embodiment 11: Assay of target affinity of the bispecific recombinant protein whose second functional binding fragment is IL12

The binding affinity of the bispecific recombinant protein to the target TIGIT and IL12 receptors was determined by flow cytometry. The following method takes LCB-018 as an example, and is suitable for the assay of the recombinant protein whose first functional antigen is TIGIT and whose second functional binding fragment is IL12A.

H_IL12 Reporter 293 cells (TIGIT+, target cells of interest) were plated in a 96-well white-walled and -bottomed transparent plate (Corning, 3903), and after the cells were adhered overnight, the medium was discarded. 200 nM of anti-TIGIT monoclonal antibody Tiragolumab or isotype control antibody was added, the plate was incubated at 37°C for 30 minutes, and the medium was discarded. 200 nM of the bispecific recombinant protein LCB-018 and 2 µg/mL of IL12B were mixed thoroughly in equal volume, the mixture was 3-fold diluted downward with 10 concentration points in total, then added to a 96-well plate as 150 µL/well, and the plate was incubated in a carbon dioxide incubator at 37°C for 6 hours. One-Glo was added, and the signal value was detected by Multomode Plate Reader (PerkinElmer, Envision 2105).

The results show that (as shown in FIG. 22), with anti-TIGIT monoclonal antibody Tiragolumab added to block TIGIT, the binding curve of the bispecific recombinant protein and the cells to be tested was obviously shifted to the right, which proved that the bispecific recombinant protein of the present disclosure using a suitable linker to connect the first functional structural fragment (the part targeting TIGIT) and the second functional structural fragment (IL12) may non-obviously improve the binding ability of the bispecific recombinant protein to the target cells. In addition, for the target cell with positive antigen, the binding affinity of the bispecific recombinant protein of the present disclosure (such as LCB-018) to this target cell (such as H_IL12 Reporter 293 cell) was about 500 times weaker than that of the IL12A and IL12B complex at the same concentration to the same cell (EC50_{LCB-018}=30 nM, EC50_{IL12A/IL12B}=0.06 nM), which also proved that the targeting effect of the bispecific recombinant protein of the present disclosure may significantly reduce the binding activity of IL12 to cells, thus reducing the potential toxic side effects of IL12, especially the toxic side effects of IL12 binding to non-target cells.

### Embodiment 12: Assay of P-STAT3 activation level in THP1 cells by the bispecific recombinant protein whose second functional binding fragment is IL10M

The assay of P-STAT3 activation levels in THP1 cells by the bispecific recombinant protein was determined by flow cytometry. The following method takes LCB-019 as an example, and is suitable for the assay of the recombinant protein whose first functional antigen is CD80 and whose second functional binding fragment is IL10M.

LCB-019, LCB-022 (control bispecific recombinant protein can not bind to CD80), Isotype, and IL10M-Fc fusion protein were diluted with a basal medium (1640) at an equimolar concentration of 106 nM for use.

Considering that LPS (sigma, L5418-2ML) can stimulate the expression of CD80 antigen on the surface of Thp1 cells (sourced from Cell Bank of Chinese Academy of Sciences), this embodiment utilized Thp1 cells stimulated by LPS for 24 hours to simulate the target CD80-positive cells (target cells of interest).

Thp1 cells (sourced from Cell Bank of Chinese Academy of Sciences) stimulated by 1 µg/mL of LPS (sigma, L5418-2ML) for 24 hours were resuspended in a basal medium (1640) at a density of 1×10⁶. The resuspended cells stimulated by LPS and Thp1 cells not stimulated by LPS were plated in a 96-well plate as a volume of 100 µL, and equal volumes of diluted LCB-019, LCB-022 (control bispecific recombinant protein), Isotype, IL10M-Fc fusion protein were added and the plate was incubated in a 37°C, 5% carbon dioxide incubator for 20 minutes. After incubation, the supernatant was removed by centrifugation, and the cells were immobilized and treated with membrane permeabilization. The cells were then resuspended in 100 µL of FACS buffer (1×PBS+2% FBS) containing 0.5 µL of PE-P-STAT3 antibody (BD, 562072) and incubated in the dark at 4°C for 1 hour. After washed twice with FACS buffer (1×PBS+2% FBS), the cells were resuspended with the addition of 200 µL of FACS buffer (1 ×PBS+2% FBS), and the P-STAT3 level was detected by FACS.

The results as shown in FIG. 23, on Thp1 cells highly expressing the antigen CD80 after LPS stimulation, the P-STAT3 level of LCB-019, a bispecific recombinant protein was basically comparable to that of IL10M-Fc fusion protein and significantly higher than that of LCB-022, a control bispecific recombinant protein without targeting function, while on Thp1 cells not stimulated by LPS, the P-STAT3 levels of LCB-019 and control bispecific recombinant protein LCB-022 were basically comparable and significantly weaker than that of IL10M-Fc fusion protein.

It can be seen that, for non-target cells with weak or no expression of the antigen, the effect of the bispecific recombinant protein on non-target cells is significantly weaker than that of IL10M-Fc fusion protein, showing its relatively higher safety profile. Whereas for the target cells with high antigen expression, the bispecific recombinant protein targeting the antigen may show a STAT3 level that is comparable to that of the IL10M-Fc fusion protein, and may fully exert and improve the efficacy of IL10M in the bispecific recombinant protein. On the contrary, the bispecific recombinant protein without the function of targeting the antigen has a relatively weak effect on the cells (non-target cells), showing a good safety profile.

### Embodiment 13: Assay of the proliferation activity of the bispecific recombinant protein whose second functional binding fragment is IL15-IL15RαSUSHI

The proliferation capability of bispecific recombinant protein on PD-1-positive hPBMCs stimulated by OKT3 for 48 hours was determined by flow cytometry. The following method takes LCB-020, LCB-021, LCB-023, and LCB-024 as examples, and is suitable for the assay of the recombinant protein whose first functional antigen is PD-1 and whose second functional binding fragment is IL15-IL15RαSUSHI.

The hPBMC cells were recovered and put into a 6-well plate pre-coated with 100 ng/mL of anti-CD3 antibody (OKT3, eBioscience, Cat. #16-0037-85), and incubated for 48 hours. Activated PBMCs were collected by centrifugation and washed once with PBS. The cells were then resuspended in a culture medium, plated into a 96-well plate at a density of 1.5E5/100 µL/well, and 1.6 nM of positive control C15Y, bispecific recombinant proteins LCB-020, LCB-021, LCB-023 and LCB-024 were added. The 96-well plate was incubated in a carbon dioxide incubator at 37°C for 96 hours. After incubation, for cell sorting, the cell membranes were first stained with anti-CD4-APC (eBioscience, Cat. #17-0049-42) and anti-CD8-FITC (Invitrogen, Cat. #MHCD0801) antibodies. After staining, the cells were permeabilized with a fixed permeabilization reagent (eBioscience^{™} Foxp3/Transcription Factor Staining Buffer Set. Invitrogen, Cat. #00-5523-00). After permeabilization, the cells were stained with anti-Ki-67-PE (Biolegend, Cat. #350504) antibody for 40 minutes. The expression of Ki-67 on CD4+ and CD8+ cell populations was analyzed by FACS at the end of staining.

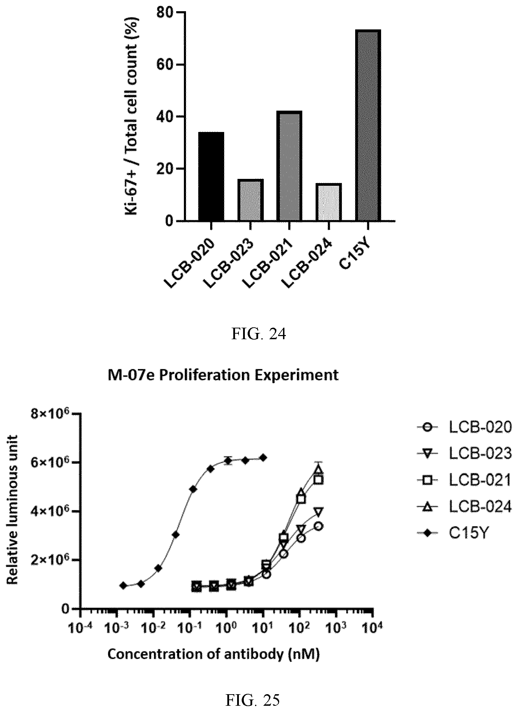

As shown in FIG. 24, all of the 5 samples have a certain pro-proliferation effect on hPBMCs with high PD-1 expression at a concentration of 1.6 nM. Among them, the positive control C15Y has the strongest activity, the pro-proliferation effects of the bispecific recombinant proteins LCB-020 and LCB-021 with the function of targeting PD-1, and the control bispecific recombinant proteins RSV×IL-15R antibodies (LCB-023, LCB-024) without the function of targeting PD-1, are weaker than that of the positive control C15Y. And the pro-proliferation effect of the bispecific recombinant proteins LCB-020 and LCB-021 with the function of targeting PD-1 is obviously stronger than that of the control bispecific recombinant proteins LCB-023 and LCB-024 without the function of targeting PD-1. At the same time, the pro-proliferation effect of the control bispecific recombinant proteins LCB-023 and LCB-024 without the function of targeting PD-1 is weak. The results show that the bispecific recombinant protein with the function of targeting the antigen may significantly enhance the pro-proliferation effect of IL15-IL15RαSUSHI on the target cells, and the pro-proliferation effect is weaker than that of the IL15-IL15RαSUSHI-Fc fusion protein (C15Y); the control bispecific recombinant protein pair (LCB-023, LCB-024) has a weak pro-proliferation effect on PD-1-positive hPBMCs stimulated by OKT3 for 24 hours, which reveals from another perspective that the bispecific recombinant protein of the present disclosure has a weak effect on non-target cells that do not express the antigen, demonstrating its high safety profile.

The proliferation activity of the PD-1-targeted bispecific recombinant protein on M-07e was determined by cell titer glo kit (Promega, Cat: G7558). The following method takes LCB-020, LCB-021, LCB-023, and LCB-024 as examples, and is suitable for the assay of the recombinant protein whose first functional antigen is PD-1 and whose second functional binding fragment is IL15-IL15RαSUSHI.

Cytokine-dependent cells M-07e were collected and washed once with PBS. The cells were diluted to a density of 2E4/50 µL with a medium not containing GM-CSF (R&D, Cat. #215-GM-050) growth factor, plated into a 96-well plate, and incubated for 4 hours for cytokine starvation. After 4 hours of starvation, serially diluted C15Y (3-fold dilutions starting from a concentration of 10 nM), bispecific recombinant proteins LCB-020, LCB-021, LCB-023, and LCB-024 (3-fold dilutions starting from a concentration of 333 nM) were added, and the plate was incubated in a CO₂ incubator at 37°C for 72 hours. After 72 hours, the number of viable cells was detected with Celltiter-glo (Promega, Cat. #G7573).

As shown in FIG. 25, the positive control C15Y exerts a very strong pro-proliferation effect at a very low concentration (EC50=0.05267 nM), while the bispecific recombinant protein of the present disclosure has to exert a pro-proliferation effect at a relatively high concentration (EC50 is about 21-37 nM), and there is no significant difference in EC50. Because M-07e do not express the target antigen of LCB-020, LCB-021, LCB-023 and LCB-024, M-07e can be regarded as a non-target cell of interest, and the bispecific recombinant protein of the present disclosure whose second functional binding fragment is IL15-IL15RαSUSHI may exert its pro-proliferation effect on non-target cells at a high concentration, the EC50 is over 400 times higher than that of the positive control.

To sum up, it can be seen that, for the pro-proliferation effect of the bispecific recombinant protein of the present disclosure on the target cells of interest with positive antigen of interest, the pro-proliferation effect of the bispecific recombinant protein with targeting function is obviously stronger than that of the bispecific recombinant protein without targeting function, but is weaker than that of the positive control C15Y; on the contrary, the pro-proliferation effect of the recombinant protein of the present disclosure shows no significant difference on non-target cells of interest with negative antigen of interest, and the effective concentration of the pro-proliferation effect is significantly higher than that of the positive control C 15Y. It can be seen that the bispecific recombinant protein of the present disclosure with targeting function may exert a pro-proliferation effect on target cells of interest at a relatively low concentration, but has to exert a pro-proliferation effect on non-target cells of interest at a very high concentration.

### Embodiment 14: Freeze-thaw stability study of the bispecific recombinant protein

The existing recombinant human albumin interferon-α 2b fusion protein has poor freeze-thaw stability and is not suitable for repeated freezing and thawing. For example, PEGylated long-acting interferon should not be frozen and shaken, and has higher requirements for transportation and storage conditions. In order to study the freeze-thaw stability of the bispecific recombinant protein of the present disclosure, repeated freeze-thaw stability tests were performed on the bispecific recombinant protein. The protein was placed in 20 mM NaAc (PH=5) buffer and subjected to 5 repeated freeze-thaw cycles under the condition of - 40°C. The purity (size exclusion chromatography, SEC) and appearance analyses were performed on samples before and after freeze-thaw. The results are shown in Table 5. LCB-011 and its mutants have good freeze-thaw stability, the purity is above 95%, and the appearance is clear after 5 repeated freeze-thaw cycles, indicating that the freeze-thaw stability of the IFN-α 2b protein part in the bispecific recombinant protein of the present disclosure is significantly better than that of IFN-α 2b monomer or PEGylated IFN-α 2b.

**Table 5: Freeze-thaw stability of bispecific recombinant proteins**

| Number | SEC-HPLC(%) | No freezing and thawing | | | Freezing and thawing for 5 times | | |
|---|---|---|---|---|---|---|---|
| | | Monomer | Multimer | Impurity with low molecular weight | Monomer | Multimer | Impurity with low molecular weight |
| 1 | LCB-011 | 96.50 | 3.50 | ND | 97.87 | 2.14 | ND |
| 2 | LCB-011-M3 | 99.11 | 0.89 | ND | 97.68 | 2.32 | ND |
| 3 | LCB-011-M4 | 98.03 | 1.97 | ND | 95.74 | 4.13 | 0.12 |

The use of any and all embodiments or exemplary language (e.g., "such as") provided herein is intended only to better illustrate the present disclosure, and does not pose a limitation on the scope of the present disclosure, unless otherwise required. No language in the specification should be construed as indicating any non-claimed element essential to the practice of the present disclosure.

All publications and patent applications cited in this specification are incorporated herein by reference in their entireties to the same extent as if each individual publication or patent application is specifically and individually indicated to be incorporated by reference. Furthermore, any theory, mechanism, demonstration or finding described herein is intended to further enhance the understanding of the present disclosure, and is not intended to limit the present disclosure in any way to such theory, mechanism, demonstration or finding. While the present disclosure has been illustrated and described in detail in the drawings and foregoing description, such illustration and description should be regarded in an illustrative rather than a restrictive sense.

Although the specific embodiments of the present disclosure have been described above, those skilled in the art should understand that these are only illustrative examples, and that various changes or modifications may be made to these embodiments without departing from the principle and essence of the present disclosure. Accordingly, the scope of protection of the present disclosure is defined by the appended claims.

## Claims

1. A bispecific recombinant protein, wherein, the bispecific recombinant protein comprises a first functional binding fragment, a second functional binding fragment, and an Fc region; the first functional binding fragment targeting an antigen of interest of the bispecific recombinant protein comprises an antigen-binding fragment, wherein a C-terminus of a CL domain or a C-terminus of a CH1 domain in the antigen-binding fragment is directly connected to or is connected via a linker to the second functional binding fragment having a function of immunoregulation and/or a function of metabolic regulation and/or a function of endocrine regulation;
preferably, the antigen-binding fragment is directly connected to or is connected via a linker to a N-terminus of the second functional binding fragment, and, a C-terminus of the second functional binding fragment is directly connected to or is connected via a linker to a N-terminus of the Fc region.

2. The bispecific recombinant protein of claim 1, wherein, the second functional binding fragment having a function of immunoregulation targets an immune checkpoint, an immune checkpoint ligand, or a cytokine receptor.

3. The bispecific recombinant protein of claim 2, wherein, the second functional binding fragment having a function of immunoregulation targets PD-1 or a ligand thereof, CD47 or a ligand thereof, CD24 or a ligand thereof, an interferon receptor, or an interleukin receptor.

4. The bispecific recombinant protein of claim 1, wherein, the second functional binding fragment having a function of metabolic regulation targets a metabolic regulator, or a metabolic regulator receptor.

5. The bispecific recombinant protein of claim 4, wherein, the second functional binding fragment having a function of metabolic regulation targets an insulin receptor, or a fibroblast growth factor receptor.

6. The bispecific recombinant protein of claim 1, wherein, the second functional binding fragment having a function of endocrine regulation targets an endocrine regulator, or an endocrine regulator receptor.

7. The bispecific recombinant protein of claim 6, wherein, the second functional binding fragment having a function of endocrine regulation targets a hormone receptor.

8. The bispecific recombinant protein of claim 1, wherein, a variable region and a constant region in the antigen-binding fragment are directly connected or are connected via a linker; or the antigen-binding fragment and the Fc region are directly connected or are connected via a linker; or both of methods mentioned above are used simultaneously to connect.

9. The bispecific recombinant protein of claim 1 or 8, wherein, the linker sequence comprises (GGGGS)n, (GGGS)n, (GGS)n, (G)n, (GS)n, (EAAAK)n, or (XP)n, n is a natural number;
preferably, n is a natural number from 0 to 5.

10. The bispecific recombinant protein of claim 2 or 3, wherein, the second functional binding fragment binds to a cytokine receptor, an immune checkpoint or an immune checkpoint ligand, the second functional binding fragment is the cytokine, the immune checkpoint ligand or a binding protein for the immune checkpoint ligand, or a functional fragment thereof or a mutant thereof; the second functional binding fragment is selected from any one of the following: an extracellular functional fragment of human SIRP family, a functional fragment of human interferon family, a functional fragment of tumor necrosis factor superfamily, a functional fragment of TGF-β superfamily, a functional fragment of interleukins, a functional fragment of chemokine family, a functional fragment of colony stimulating factor family, a functional fragment of growth factors, or a mutant thereof.

11. The bispecific recombinant protein of claim 10, wherein, the second functional binding fragment is an extracellular D1 domain of human SIRPα or a mutant thereof.

12. The bispecific recombinant protein of claim 10, wherein, the interferon receptor is a type I or type II human interferon receptor, preferably a human interferon γ (IFN-γ) receptor or a human interferon β (IFN-β) receptor.

13. The bispecific recombinant protein of claim 10, wherein, the second functional binding fragment is an interleukin, a truncated variant thereof, or a mutant thereof.

14. The bispecific recombinant protein of claim 13, wherein, the interleukin is an immunoregulatory factor or chemokine selected from any one of the following: IL-1 family, IL-2 family, IL-3 family, IL-6 family, IL-8 family, IL-10 family, IL-12 family, and IL-17 family.

15. The bispecific recombinant protein of any one of claims 1-14, wherein, the first functional binding fragment targets any one or more of the following targets: 5T4, AGS-16, ALK1, ANG-2, B7-H3, B7-H4, c-fms, c-Met, CA6, CD123, CD19, CD20, CD22, CD24, EpCAM, CD30, CD32b, CD37, CD38, CD40, CD52, CD70, CD71, CD74, CD79b, CD80, CD83, CD86, CD98, CD206, CEA, CEACAM5, CLDN18.2, CLDN6, CS1, CCR5, CXCR4, DLL-4, EGFR, EGFRvIII, EGP-1, ENPP3, EphA3, ETBR, FGFR2, FN, FR-α, GCC, GD2, GPC-3, GPNMB, HER2, HER3, HLA-DR, ICAM-1, IGF-1R, IL-3R, LIV-1, MSLN, MUC16, MUC1, NaPi2b, Nectin-4, Notch 2, Notch 1, PD-1, PD-L1, PD-L2, PDGFR-α, PS, PSMA, SLTRK6, STEAP1, TEM1, TIGIT, VEGFR, CD25, CD27L, DKK-1, CSF-1R, MSB0010718C, BCMA, CD138, TROP2, Siglec15, CD155, and AFP;
preferably, the second functional binding fragment comprises an extracellular D1 domain of human SIRPα or a mutant thereof, a human interferon β or a human interferon γ, or an interleukin, a truncated variant thereof or a mutant thereof, the first functional binding fragment targets a tumor cell or an immune cell.

16. The bispecific recombinant protein of any one of claims 1-14, wherein, the bispecific recombinant protein consists of a chain A and a chain B, the chain A binds to the chain B by intermolecular force, by covalent bond, or by salt bond, or by a combination of two or three of binding methods mentioned above.

17. The bispecific recombinant protein of claim 16, wherein, the Fc region comprises an Fc region native sequence or an Fc region non-native sequence.

18. The bispecific recombinant protein of claim 17, wherein, the Fc region is a human Fc region;
preferably, the Fc region of the chain A binds to the Fc region of the chain B by knobs-into-holes; and/or, the Fc region is an Fc region of human IgG;
more preferably, the Fc region is an Fc region of human IgG1 or IgG4.

19. The bispecific recombinant protein of claim 17 or 18, wherein, the C-terminus of the CL domain, the C-terminus of the CH1 domain or the C-terminus of the second functional binding fragment is directly connected to the Fc region or is connected via a linker to the Fc region.

20. The bispecific recombinant protein of claim 17 or 18, wherein, the first functional binding fragment is any one or more of antigen-binding fragments targeting the following targets: 5T4, AGS-16, ALK1, ANG-2, B7-H3, B7-H4, c-fms, c-Met, CA6, CD123, CD19, CD20, CD22, CD24, EpCAM, CD30, CD32b, CD37, CD38, CD40, CD52, CD70, CD71, CD74, CD79b, CD80, CD83, CD86, CD98, CD206, CEA, CEACAM5, CLDN18.2, CLDN6, CS1, CCR5, CXCR4, DLL-4, EGFR, EGFRvIII, EGP-1, ENPP3, EphA3, ETBR, FGFR2, FN, FR-α, GCC, GD2, GPC-3, GPNMB, HER2, HER3, HLA-DR, ICAM-1, IGF-1R, IL-3R, LIV-1, MSLN, MUC16, MUC1, NaPi2b, Nectin-4, Notch 2, Notch 1, PD-1, PD-L1, PD-L2, PDGFR-α, PS, PSMA, SLTRK6, STEAP1, TEM1, TIGIT, VEGFR, CD25, CD27L, DKK-1, CSF-1R, MSB0010718C, BCMA, CD138, TROP2, Siglec15, CD155, and AFP; the antigen-binding fragment is a human-mouse chimeric antigen-binding fragment, a humanized antigen-binding fragment, or a fully human antigen-binding fragment.

21. The bispecific recombinant protein of claim 20, wherein,
when the first functional binding fragment targets CD20, EGFR, EGFRvIII, PD-L1, PD-L2, HER2, HER3, CD138, CD44, CD24, EpCAM, CLDN18.2, CD38, BCMA, MUC1, or TROP2, the second functional binding fragment comprises an extracellular D1 domain of SIRPα or a mutant thereof;
when the first functional binding fragment targets TIGIT, Siglec15, PD-1, PD-L1, PD-L2, CD71, CD80, CD86, CD206, or CCR5, the second functional binding fragment comprises IFN-β, IFN-γ, IL-10M, IL-12A, or a complex formed by IL-15 and IL15RαSUSHI, or a mutant thereof;
preferably:
the first functional binding fragment targets CD20, EpCAM, CD24, or EGFR, the second functional binding fragment comprises an extracellular D1 domain of SIRPα or a mutant thereof; preferably, amino acid sequence of the second functional binding fragment is as shown in SEQ ID NO: 50; more preferably, amino acid sequence of the chain A is as shown in SEQ ID NO: 1, amino acid sequence of the chain B is as shown in SEQ ID NO: 2 or 3; amino acid sequence of the chain A is as shown in SEQ ID NO: 61, amino acid sequence of the chain B is as shown in SEQ ID NO: 62; amino acid sequence of the chain A is as shown in SEQ ID NO: 27, amino acid sequence of the chain B is as shown in SEQ ID NO: 28; amino acid sequence of the chain A is as shown in SEQ ID NO: 29, amino acid sequence of the chain B is as shown in SEQ ID NO: 30; amino acid sequence of the chain A is as shown in SEQ ID NO: 56, amino acid sequence of the chain B is shown in SEQ ID NO: 57;
the first functional binding fragment targets TIGIT, CD80 or PD-1, the second functional binding fragment comprises IL-12A, an IL-10 monomer mutant, or a complex formed by IL15 and IL-15RαSUSHI, amino acid sequences of the IL-12A, IL-10 monomer mutant, and IL15 and IL-15RαSUSHI are as shown in SEQ ID NOs: 51, 52, 53, and 54, respectively, or a mutant of the second functional binding fragment mentioned above; preferably, amino acid sequence of the chain A is as shown in SEQ ID NO:35, amino acid sequence of the chain B is as shown in SEQ ID NO:36; amino acid sequence of the chain A is as shown in SEQ ID NO:37, amino acid sequence of the chain B is as shown in SEQ ID NO: 38; amino acid sequence of the chain A is as shown in SEQ ID NO: 39, amino acid sequence of the chain B is as shown in SEQ ID NO: 40 or 41;
the first functional binding fragment targets CD38 or AFP, the second functional binding fragment comprises an extracellular D1 domain of SIRPα or a mutant thereof, or IFN-β or a mutant thereof; preferably, amino acid sequence of the second functional binding fragment is as shown in SEQ ID NO: 50 or 55; more preferably, amino acid sequence of the chain A is as shown in SEQ ID NO: 31, amino acid sequence of the chain B is as shown in SEQ ID NO: 32; amino acid sequence of the chain A is as shown in SEQ ID NO: 33, amino acid sequence of the chain B is as shown in SEQ ID NO: 34; or, amino acid sequence of the chain A is as shown in SEQ ID NO: 58, amino acid sequence of the chain B is as shown in SEQ ID NO: 60.

22. A nucleic acid molecule encoding the bispecific recombinant protein of any one of claims 1-21; wherein, a nucleic acid molecule encoding the first functional binding fragment and a nucleic acid encoding the second functional binding fragment are in a same DNA strand, or a nucleic acid molecule encoding the first functional binding fragment and a nucleic acid encoding the second functional binding fragment are in different DNA strands;
preferably, a nucleic acid molecule encoding the Fc region is in a same DNA strand with the nucleic acid encoding the first functional binding fragment or the second functional binding fragment, a nucleic acid molecule encoding the Fc region is in different DNA strands with the nucleic acid encoding the first functional binding fragment or the second functional binding fragment.

23. An expression vector comprising the nucleic acid molecule of claim 22.

24. A host cell transformed with the expression vector of claim 23.

25. A method for preparing the bispecific recombinant protein of any one of claims 1-21, which comprises transforming the host cell of claim 24 with the expression vector of claim 23, the host cell is cultured under a condition suitable for expression, and then the bispecific recombinant protein is obtained by expression.

26. A medicament or pharmaceutical composition, wherein, the medicament or pharmaceutical composition comprises the bispecific recombinant protein of any one of claims 1-21.

27. A use of the bispecific recombinant protein of any one of claims 1-21 in the manufacture of a medicament for the treatment of tumors, autoimmune diseases, infectious diseases, sepsis, graft-versus-host diseases, metabolic disorders, endocrine disorders.

28. The use of claim 27, wherein, the tumor is a solid tumor or a hematological tumor; preferably, the solid tumor is selected from any one of the following: breast cancer, colorectal cancer, lung cancer, pancreatic cancer, esophagus cancer, endometrial cancer, ovarian cancer, gastric cancer, prostate cancer, renal cancer, cervical cancer, thyroid cancer, uterine cancer, bladder cancer, neuroendocrine cancer, head and neck cancer, liver cancer, nasopharyngeal cancer, testicular cancer, small cell lung cancer, non-small cell lung cancer, melanoma, basal cell carcinoma, squamous cell carcinoma, dermatofibrosarcoma protuberan, Merkel cell carcinoma, glioblastoma, glioma, sarcoma, mesothelioma, and myelodysplastic syndrome; the hematological tumor is selected from myeloma, lymphoma or leukemia;
the autoimmune disease is selected from any one of the following: Hashimoto's thyroiditis, type 1 diabetes, systemic lupus erythematosus, rheumatoid arthritis, and sjogren syndrome;
the infectious disease is selected from any one of the following: viral infections, bacterial infections, fungal infections, and other pathogenic infections.
